# EUROPEAN PATENT APPLICATION

(11) **EP 2 174 649 A1**
(43) Date of publication of application: **14.04.2010**
(21) Application number: 10150347.2
(22) Date of filing: 24.09.2004
(51) Int. Cl.: A61K 8/97, A61Q 11/00

(54) **Morinda Citrifolia-Based Oral Care Compositions and Methods**

(30) Priority: 26.09.2003 US 506683 P; 23.09.2004 US 948815
(62) Divisional of application: 04789060.3
(71) Applicant: Tahitian Noni International, Inc, Provo, UT 84604 (US)
(72) Inventor: Jensen, Claude Jarakae, Cedar Hills UT 84062 (US); Palu, Afa K, Orem UT 84058 (US); Zhou, Bing-Nan, Pleasant Grove UT 84062 (US); Isami, Fumiyuki, Kotoku Tokyo 135-0001 (JP); Ogden, Robert V, Cedar Hills, UT 84062 (US); Story, Stephen P, Alpine UT 84004 (US)
(74) Representative: Gemmell, Peter Alan

(57) **Abstract**

A composition for use in treating tooth decay comprises:
an effective amount of processed *Morinda citrifolia* product wherein said *Morinda citrifolia* is selected from the group consisting of *Morinda citrifolia* fruit, *Morinda citrifolia* fruit extract, *Morinda citrifolia* fruit juice concentrate, *Morinda citrifolia* oil, *Morinda citrifolia* leaf powder, and *Morinda citrifolia* leaf extract; and
a carrier.

## Description

### 1. Field of the Invention

The present invention relates to an oral care composition and, more particularly, to an oral care composition for promoting healthy teeth and gums comprised of components of the Indian Mulberry plant, scientifically known as *Morinda citrifolia L.*

### 2. Background and Related Art

Teeth are the only mammalian body tissue not subject to metabolic turnover, thus rendering them almost indestructible. Despite this, teeth are constantly subjected to bacterial attack, which may cause decalcification of tooth enamel and erosion of surrounding oral tissues over time.

Teeth are hard, calcified structures embedded in the bone of the jaws of vertebrates that perform the primary function of mastication. Humans and most other mammals have a temporary set of teeth, the deciduous, or milk, teeth; in humans, they usually erupt between the 6th and 24th months. These number 20 in all: 2 central incisors, 2 lateral incisors, 2 canines, and 4 premolars in each jaw. At about six years of age, the preliminary teeth begin to be shed as the permanent set replaces them. The last of the permanent teeth (wisdom teeth) may not appear until the 25th year, and in some persons do not erupt at all. The permanent teeth generally number 32 in all: 4 incisors, 2 canines, 4 bicuspids, and 4 (or 6, if wisdom teeth develop) molars in each jaw. Human canines are the smallest found in any mammal. Among all mammals, the tooth consists of a crown, the portion visible in the mouth, and one or more roots embedded in a gum socket. The portion of the gum surrounding the root, known as the periodontal membrane, cushions the tooth in its bony socket. The jawbone serves as a firm anchor for the root. The center of the crown is filled with soft, pulpy tissue containing blood vessels and nerves; this tissue extends to the tip of the root by means of a canal. Surrounding the pulp and making up the greater bulk of the tooth is a hard, bony substance, dentin. The root portion has an overlayer of cementum, while the crown portion has an additional layer of enamel, the hardest substance in the body.

Proper diet is necessary for the development and maintenance of sound teeth, especially sufficient calcium, phosphorus, and vitamins D and C. The most common disorder that affects the teeth is dental caries (tooth decay). A widely accepted explanation of the process of tooth decay is that salivary bacteria convert carbohydrate particles in the mouth into lactic acid, which attacks the enamel, dentin, and, if left untreated, the pulp of the teeth. Regular cleansing and semiannual dental examinations are important in preventing dental caries and gum disorders. Fluoridation of public water supplies and use of fluoride toothpastes also help prevent caries.
Periodontal disease, including gingivitis, is an infection of the tissues that surround and support the teeth, including the gums, periodontal ligament and alveolar bone. Although several factors may aggravate periodontal disease, the primary cause of periodontal disease is linked to bacteria contained within dental plaque.

Plaque is a sticky, colorless film of bacteria and sugars that constantly forms on teeth. Plaque causes cavities when the acids from plaque attack teeth after eating, eventually causing the tooth enamel to break down, resulting in tooth decay and halitosis. If left untreated, periodontal disease may develop, eventually leading to tooth loss, as well as contributing to conditions such as stroke, diabetes, premature births, heart disease and respiratory disease.

Microorganisms have an essential role in the development of caries, endodontic and periodontal diseases that can destroy oral tissues. A 1999 report indicated that more than 50% of all children, 85% of all adults aged over 18 years, and more than 50% of the elderly aged over 75 years suffer from caries lesions. The caries from active lesions must often be removed to prevent the progressive decay of remaining tooth structure. Caries removal is the primary reason for restoring teeth. Secondary caries is also directly responsible for 50% of all restoration failures. The net effect of dental caries in the U.S. is that 90 million restorations must be replaced, and a further 200 million restorations must be placed each year. Additionally, uncounted millions of teeth are extracted, and 15 million teeth undergo root canal therapy each year in the U.S. In the U.S. alone, approximately $60 billion dollars per year is spent on professional dental treatments, $172 billion on medical products, $122 billion on prescription drugs, and $50 billion on other medical products. In the rest of the world it is estimated that over $200 billion dollars are spent on professional dental treatments.

A substantial number of bacterial species have been identified as inhabitants of the oral cavity. However, because of bacterial interactions, nutrient availability and low oxygen potentials in root canals with necrotic pulp, the number of bacterial species present in endodontic infections are restricted. These selective conditions lead to the predominance of anaerobic microorganisms that survive and multiply, causing infections that stimulate local bone resorption, and are more resistant to endodontic treatment. Cleaning is one of the main objectives of root-canal preparation. Thorough cleaning removes microorganisms and permits better adaption of filling materials and enhances the action of intracanal medicaments. The choice of an irrigant is of great importance because they act as lubricants during instrumentation, flush debris and bacteria out of the canal, and react with pulp, necrotic tissues and microorganisms and their subproducts. Sodium hypochlorite has been extensively used for several decades for this purpose. Its excellent properties of tissue dissolution and antimicrobial activity make it the irrigant of choice for the treatment of teeth with pulp necrosis, even though it has several undesirable characteristics such as tissue toxicity at high concentrations, risk of emphysema when overfilling, allergic potential and disagreeable smell and taste. Moreover, Sodium hypochlorite does not totally clean the surfaces of the walls. Chlorhexidine has been studied for its various properties; including antimicrobial activity and biocompatibility with the objective of being an alternative to sodium hypochlorite. However, its capacity to clean root canal walls was recently found to be inferior to sodium hypochlorite. These problems suggest that Sodium hypochlorite and other alternative medicaments including chlorhexidine are not fully optimized; and new irrigants should be evaluated for use as dental medicaments.

The ideal irrigant should have an antimicrobial action, low toxicity and good biocompatibility to oral tissues, and have the capacity to clean the walls of the root canal and remove the smear layer. The smear layer is a 1 mm thick layer of denatured cutting debris produced on instrumented cavity surfaces, and is composed of dentin, odontodiastic processes, non-specific inorganic contaminants and microorganisms. The removal of smear layer from the instrumented root canal walls is controversial. Its removal provides better sealing of the endodontic filling material to dentin, and will avoid the leakage of microorganisms into oral tissues. The infiltration of microorganisms into oral tissues must be prevented because these often cause complications leading to treatment failure.

Thus, while improvements in alternatives for treating patients with periodontal disease and other oral and dental disorders have occurred in recent decades, researchers are continually attempting to obtain improved methods of treatment, as some of the treatments are undesirable. Accordingly, it would be an improvement in the art to augment or even replace the treatments currently used with other treatments to provide increased results in treating oral and dental diseases and disorders. Such treatment methods and compositions are disclosed and claimed herein.

### SUMMARY OF THE INVENTION

The present invention relates to an oral care composition and, more particularly, to an oral care composition for promoting healthy teeth and gums comprised of components of the Indian Mulberry plant, scientifically known as *Morinda citrifolia L. Morinda citrifolia* has beneficial antimicrobial properties while being biocompatible and therefore presents significant medicinal potential as part of dental treatment.

Certain embodiments of the present invention utilize components of the Indian Mulberry plant, scientifically known as *Morinda citrifolia L*., to treat one or more oral and dental disorders, including periodontal diseases such as gingivitis and periodontitis, tooth decay, halitosis, and other mouth irritations. The present invention incorporates components of the *Morinda citrifolia* plant as constituents of oral care compositions to treat and prevent various oral and dental-related disorders. Certain embodiments of the present invention comprise an excipient base component and an active component comprising *Morinda citrifolia* in an amount up to fifty percent by weight.

In view of the foregoing, certain embodiments of the present invention provide an oral dental formulation capable of boosting immune response to gum diseases or tooth decay.

Furthermore, certain embodiments of the present invention provide an oral dental formulation and method of reducing gingival bleeding by reducing inflammation in the gingival sulcus and increasing gingival collagen density.

Certain embodiments of the present invention provide an oral dental formulation and method of reducing periodontal attachment loss by reducing plaque formation and/or plaque adhesion to teeth and surrounding oral tissues.

Also, certain embodiments of the present invention provide an oral dental formulation and method to reduce epithelial cell wall permeability to bacterial toxins, and further to reduce collagenase enzyme activity in the presence of bacteria.

It is yet another object of certain embodiments of the present invention to provide an oral dental formulation and method to increase cellular respiration and oxygen saturation of cells in the mouth.

Certain embodiments of the present invention provide an oral dental formulation and method to reduce the incidence and duration of apthus ulcersin the mouth.

Certain embodiments of the present invention provide an oral dental formulation and method to clean teeth, remove surface staining from teeth, and whiten teeth.

Certain embodiments of the present invention provide an oral dental formulation and method to treat and prevent gum diseases and tooth decay in mammals and further to generally enhance overall dental health.

In addition, certain embodiments of the present invention provide an oral dental formulation and method to reduce halitosis in mammals.

These and other features and advantages of the present invention will be set forth or will become more fully apparent in the description that follows and in the appended claims. The features and advantages may be realized and obtained by means of the instruments and combinations particularly pointed out in the appended claims. Furthermore, the features and advantages of the invention may be learned by the practice of the invention or will be obvious from the description, as set forth hereinafter.

### DETAILED DESCRIPTION OF THE INVENTION

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes that come within the meaning and range of equivalency of the claims are to be embraced within their scope.

As used in this specification, the term "oral disorder" or "dental disorder" refers to any type of disease, condition, attribute or disorder that affects any one of the teeth, gums and surrounding oral tissues. Examples of such oral or dental disorders include periodontal diseases such as gingivitis and periodontitis, tooth decay, halitosis, mouth irritations and lesions, and other conditions affecting the teeth, gums and/or surrounding oral tissues. The term "*Morinda citrifolia*" refers to any component of the *Morinda citrifolia (L.)* plant, including juice of the *Morinda citrifolia* fruit, its extracts, fruit juice concentrates, its oil, leaves, leaf powder, leaf extracts, bark, bark extracts, root, root extract, root bark, and root bark extracts. The term "Tahitian Noni^{®}" Juice refers to a product that includes processed components from the *Morinda citrifolia L* plant. In one embodiment, Tahitian Noni^{®} Juice includes reconstituted *Morinda citrifolia L.* fruit juice from pure juice puree of French Polynesia. Tahitian Noni^{®} Juice may also include other natural juices, such as a natural grape juice concentrate, a natural blueberry juice concentrate, and/or another natural juice concentrate. In a further embodiment, Tahitian Noni^{®} Juice is processed from dried or powdered *Morinda citrifolia L.* Tahitian Noni^{®} Juice may be obtained from Morinda, Inc., which has a principal place of business located at 5152 N. Edgewood Dr. #100, Provo, UT, 84604.

As used herein, an "effective amount" is an amount sufficient to effect beneficial or desired results. An effective amount can be administered in one or more administrations, applications or dosages. For example, an effective amount of *Morinda citrifolia* is an amount sufficient to reduce dental plaque, suppress bacterial growth, and reduce adhesiveness of plaque, to thereby inhibit formation of dental caries. Such effective amounts can be determined without undue experimentation by those skilled in the art.

In accordance with the present invention, components of the *Morinda citrifolia (L.)* plant may be used in combination with an excipient base to treat and/or prevent an oral or dental disorder. Certain embodiments of the present invention may be in the form of a dietary supplement, a topically applied oral dental formulation, or any other form known to those in the art.

The following disclosure of the present invention is grouped into three subheadings, namely "General Discussion of *Morinda citrifolia* and the Methods Used to Produce Processed *Morinda citrifolia* Products," "Formulations and Methods of Administration" and "Oral Care" The utilization of the subheadings is for convenience of the reader only and is not to be construed as limiting in any sense.

It will be readily understood that the elements of the present invention, as generally described and illustrated in the figures herein, could be combined and used in a wide variety of different formulations and methods. Thus, the following more detailed description of the embodiments of the system and method of the present invention is not intended to limit the scope of the invention, as claimed, but is merely representative of the presently preferred embodiments of the invention.

### 1. General Discussion of Morinda citrifolia and the Methods Used to Produce Processed Morinda citrifolia Products

The Indian Mulberry or Noni plant, known scientifically as *Morinda Citrifolia L.* ("*Morinda citrifolia* "), is a shrub or small tree up to 10 m in height. The leaves are oppositely arranged with an elliptic to ovate form. The small white flowers are contained in a fleshy, globose, head-like cluster. The fruits are large, fleshy, and ovoid. At maturity, they are creamy-white and edible, but have an unpleasant taste and odor. The plant is native to Southeast Asia and has spread in early times to a vast area from India to eastern Polynesia. It grows randomly in the wild, and it has been cultivated in plantations and small individual growing plots. The *Morinda citrifolia* flowers are small, white, three to five lobed, tubular, fragrant, and about 1.25 cm long. The flowers develop into compound fruits composed of many small drupes fused into an ovoid, ellipsoid or roundish, lumpy body, with waxy, white, or greenish-white or yellowish, semi-translucent skin. The fruit contains "eyes" on its surface, similar to a potato. The fruit is juicy, bitter, dull-yellow or yellowish-white, and contains numerous red-brown, hard, oblong-triangular, winged 2-celled stones, each containing four seeds.

When fully ripe, the fruit has a pronounced odor like rancid cheese. Although the fruit has been eaten by several nationalities as food, the most common use of the *Morinda citrifolia* plant was as a red and yellow dye source. Recently, there has been an interest in the nutritional and health benefits of the *Morinda citrifolia* plant, further discussed below.

Because the *Morinda citrifolia* fruit is for all practical purposes inedible, the fruit must be processed in order to make it palatable for human consumption and included in the nutraceutical used to promote oral care. Processed *Morinda citrifolia* fruit juice can be prepared by separating seeds and peels from the juice and pulp of a ripened *Morinda citrifolia* fruit; filtering the pulp from the juice; and packaging the juice. Alternatively, rather than packaging the juice, the juice can be immediately included as an ingredient in another food product, frozen or pasteurized. In some embodiments, the juice and pulp can be pureed into a homogenous blend to be mixed with other ingredients. Other process include freeze-drying the fruit and juice. The fruit and juice can be reconstituted during production of the final juice product. Still other processes include air-drying the fruit and juices, prior to being masticated.

The present invention also contemplates the use of fruit juice and/or puree fruit juice extracted from the *Morinda Citrifolia* plant. In a currently preferred process of producing *Morinda citrifolia* fruit juice, the fruit is either hand picked or picked by mechanical equipment. The fruit can be harvested when it is at least one inch (2-3 cm) and up to 12 inches (24-36 cm) in diameter. The fruit preferably has a color ranging from a dark green through a yellow-green up to a white color, and gradations of color in between. The fruit is thoroughly cleaned after harvesting and before any processing occurs.

The fruit is allowed to ripen or age from 0 to 14 days, with most fruit being held from 2 to 3 days. The fruit is ripened or aged by being placed on equipment so it does not contact the ground. It is preferably covered with a cloth or netting material during aging, but can be aged without being covered. When ready for further processing the fruit is light in color, from a light green, light yellow, white or translucent color. The fruit is inspected for spoilage or for excessively green color and hard firmness. Spoiled and hard green fruit is separated from the acceptable fruit.

The ripened and aged fruit is preferably placed in plastic lined containers for further processing and transport. The containers of aged fruit can be held from 0 to 120 days. Most fruit containers are held for 7 to 14 days before processing. The containers can optionally be stored under refrigerated conditions or ambient/room temperature conditions prior to further processing. The fruit is unpacked from the storage containers and is processed through a manual or mechanical separator. The seeds and peel are separated from the juice and pulp.

The juice and pulp can be packaged into containers for storage and transport. Alternatively, the juice and pulp can be immediately processed into a finished juice product. The containers can be stored in refrigerated, frozen, or room temperature conditions.

The *Morinda citrifolia* juice and pulp are preferably blended in a homogenous blend, after which they may be mixed with other ingredients, such as flavorings, sweeteners, nutritional ingredients, botanicals, and colorings. The finished juice product is preferably heated and pasteurized at a minimum temperature of 181°F (83°C) or higher up to 212°F (100°C).

Another product manufactured is *Morinda citrifolia* puree and puree juice, in either concentrate or diluted form. Puree is essentially the pulp separated from the seeds and is different from the fruit juice product described herein.

Each product is filled and sealed into a final container of plastic, glass, or another suitable material that can withstand the processing temperatures. The containers are maintained at the filling temperature or may be cooled rapidly and then placed in a shipping container. The shipping containers are preferably wrapped with a material and in a manner to maintain or control the temperature of the product in the final containers.

The juice and pulp may be further processed by separating the pulp from the juice through filtering equipment. The filtering equipment preferably consists of, but is not limited to, a centrifuge decanter, a screen filter with a size from 0.01 micron up to 2000 microns, more preferably less than 500 microns, a filter press, reverse osmosis filtration, and any other standard commercial filtration devices. The operating filter pressure preferably ranges from 0.1 psig up to about 1000 psig. The flow rate preferably ranges from 0.1 g.p.m. up to 1000 g.p.m., and more preferably between 5 and 50 g.p.m. The wet pulp is washed and filtered at least once and up to 10 times to remove any juice from the pulp. The wet pulp typically has a fiber content of 10 to 40 percent by weight. The wet pulp is preferably pasteurized at a temperature of 181°F (83°C) minimum and then packed in drums for further processing or made into a high fiber product.

The processed *Morinda citrifolia* product may also exist as a dietary fiber. Still further, the processed *Morinda citrifolia* product may also exist in oil form. The *Morinda citrifolia* oil typically includes a mixture of several different fatty acids as triglycerides, such as palmitic, stearic, oleic, and linoleic fatty acids, and other fatty acids present in lesser quantities. In addition, the oil preferably includes an antioxidant to inhibit spoilage of the oil. Conventional food grade antioxidants are preferably used.

Drying may further process the wet pulp. The methods of drying may include freeze-drying, drum drying, tray drying, sun drying, and spray drying. The dried *Morinda citrifolia* pulp may include a moisture content in the range from 0.1 to 15 percent by weight and more preferably from 5 to 10 percent by weight. The dried pulp preferably has a fiber content in the range from 0.1 to 30 percent by weight, and more preferably from 5 to 15 percent by weight.

The high fiber product may include wet or dry *Morinda citrifolia* pulp, supplemental fiber ingredients, water, sweeteners, flavoring agents, coloring agents, and/or nutritional ingredients. The supplemental fiber ingredients may include plant based fiber products, either commercially available or developed privately. Examples of some typical fiber products are guar gum, gum arabic, soybean fiber, oat fiber, pea fiber, fig fiber, citrus pulp sacs, hydroxymethylcellulose, cellulose, seaweed, food grade lumber or wood pulp, hemicellulose, etc. Other supplemental fiber ingredients may be derived from grains or grain products. The concentrations of these other fiber raw materials typically range from 0 up to 30 percent, by weight, and more preferably from 10 to 30 percent by weight.

Typical sweeteners may include, but are not limited to, natural sugars derived from corn, sugar beet, sugar cane, potato, tapioca, or other starch-containing sources that can be chemically or enzymatically converted to crystalline chunks, powders, and/or syrups. In addition, sweeteners can consist of artificial or high intensity sweeteners, some of which are aspartame, sucralose, stevia, saccharin, etc. The concentration of sweeteners may be between from 0 to 50 percent by weight, of the formula, and more preferably between about 1 and 5 percent by weight.

Typical flavors can include, but are not limited to, artificial and/or natural flavor or ingredients that contribute to palatability. The concentration of flavors may range, for example, from 0 up to 15 percent by weight, of the formula. Colors may include food grade artificial or natural coloring agents having a concentration ranging from 0 up to 10 percent by weight, of the formula.

Typical nutritional ingredients may include vitamins, minerals, trace elements, herbs, botanical extracts, bioactive chemicals and compounds at concentrations from 0 up to 10 percent by weight. Examples of vitamins one can add to the fiber composition include, but are not limited to, vitamins A, B1 through B12, C, D, E, Folic Acid, Pantothenic Acid, Biotin, etc. Examples of minerals and trace elements one can add to the fiber composition include, but are not limited to, calcium, chromium, copper, cobalt, boron, magnesium, iron, selenium, manganese, molybdenum, potassium, iodine, zinc, phosphorus, etc. Herbs and botanical extracts include, but are not limited to, alfalfa grass, bee pollen, chlorella powder, Dong Quai powder, Ecchinacea root, Gingko Biloba extract, Horsetail herb, Indian mulberry, Shitake mushroom, spirulina seaweed, grape seed extract, etc. Typical bioactive chemicals may include, but are not limited to, caffeine, ephedrine, L-carnitine, creatine, lycopene, etc.

The juice and pulp can be dried using a variety of methods. The juice and pulp mixture can be pasteurized or enzymatically treated prior to drying. The enzymatic process begins with heating the product to a temperature between 75°F and 135°F. It is then treated with either a single enzyme or a combination of enzymes. These enzymes include, but are not limited to, amylase, lipase, protease, cellulase, bromelin, etc. The juice and pulp may also be dried with other ingredients, such as those described above in connection with the high fiber product. The typical nutritional profile of the dried juice and pulp is 1 to 20 percent moisture, 0.1 to 15 percent protein, 0.1 to 20 percent fiber, and the vitamin and mineral content.

The filtered juice and the water from washing the wet pulp are preferably mixed together. The filtered juice may be vacuum evaporated to a brix of 40 to 70 and a moisture of 0.1 to 80 percent, more preferably from 25 to 75 percent. The resulting concentrated *Morinda citrifolia* juice may or may not be pasteurized. For example, the juice would not be pasteurized in circumstances where the sugar content or water activity was sufficiently low enough to prevent microbial growth. It is packaged for storage, transport and/or further processing.

In accordance with the present invention, an oral care composition in the form of a dietary supplement or topically applied oral dental formulation or other form is used to treat and/or prevent one or more oral or dental disorders. The amount used per treatment may depend on various factors, including the type of oral or dental disorder, the physical characteristics of the patient, etc.

The *Morinda citrifolia* plant is rich in natural ingredients. Those ingredients that have been discovered include: (from the leaves): alanine, anthraquinones, arginine, ascorbic acid, aspartic acid, calcium, beta-carotene, cysteine, cystine, glycine, glutamic acid, glycosides, histidine, iron, leucine, isoleucine, methionine, niacin, phenylalanine, phosphorus, proline, resins, riboflavin, serine, beta-sitosterol, thiamine, threonine, tryptophan, tyrosine, ursolic acid, and valine; (from the flowers): acacetin-7-o-beta-d(+)-glucopyranoside, 5,7-dimethyl-apigenin-4'-o-beta-d(+)-galactopyranoside, and 6,8-dimethoxy-3-methylanthraquinone-1-o-beta-rhamnosyl-glucopyranoside; (from the fruit): acetic acid, asperuloside, butanoic acid, benzoic acid, benzyl alcohol, 1-butanol, caprylic acid, decanoic acid, (E)-6-dodeceno-gamma-lactone, (Z,Z,Z)-8,11,14-eicosatrienoic acid, elaidic acid, ethyl decanoate, ethyl hexanoate, ethyl octanoate, ethyl palmitate, (Z)-6-(ethylthiomethyl) benzene, eugenol, glucose, heptanoic acid, 2-heptanone, hexanal, hexanamide, hexanedioic acid, hexanoic acid (hexoic acid), 1-hexanol, 3-hydroxy-2-butanone, lauric acid, limonene, linoleic acid, 2-methylbutanoic acid, 3-methyl-2-buten-1-ol, 3-methyl-3-buten-1-ol, methyl decanoate, methyl elaidate, methyl hexanoate, methyl 3-methylthio-propanoate, methyl octanoate, methyl oleate, methyl palmitate, 2-methylpropanoic acid, 3-methylthiopropanoic acid, myristic acid, nonanoic acid, octanoic acid (octoic acid), oleic acid, palmitic acid, potassium, scopoletin, undecanoic acid, (Z,Z)-2,5-undecadien-l-ol, and vomifol; (from the roots): anthraquinones, asperuloside (rubichloric acid), damnacanthal, glycosides, morindadiol, morindine, morindone, mucilaginous matter, nor-damnacanthal, rubiadin, rubiadin monomethyl ether, resins, soranjidiol, sterols, and trihydroxymethyl anthraquinone-monomethyl ether; (from the root bark): alizarin, chlororubin, glycosides (pentose, hexose), morindadiol, morindanigrine, morindine, morindone, resinous matter, rubiadin monomethyl ether, and soranjidiol; (from the wood): anthragallol-2,3-dimethylether; (from the tissue culture): damnacanthal, lucidin, lucidin-3-primeveroside, and morindone-6beta-primeveroside; (from the plant): alizarin, alizarin-alpha-methyl ether, anthraquinones, asperuloside, hexanoic acid, morindadiol, morindone, morindogenin, octanoic acid, and ursolic acid. The present invention contemplates utilizing all parts of the *M citrifolia* plant alone, in combination with each other or in combination with other ingredients. The above listed portions of the M *citrifolia* plant is not an exhaustive list of parts of the plant to be used but are merely exemplary. Thus, while some of the parts of the *M. citrifolia* plant are not mentioned above (e.g., seed from the fruit, the pericarp of the fruit, the bark or the plant) the present invention contemplates the use of all of the parts of the plant.

Recently, as mentioned, many health benefits have been discovered stemming from the use of products containing *Morinda citrifolia.* One benefit of *Morinda citrifolia* is found in its ability to isolate and produce Xeronine, which is a relatively small alkaloid physiologically active within the body. Xeronine occurs in practically all healthy cells of plants, animals and microorganisms. Even though *Morinda citrifolia* has a negligible amount of free Xeronine, it contains appreciable amounts of the precursor of Xeronine, called Proxeronine. Further, *Morinda citrifolia* contains the inactive form of the enzyme Proxeronase that releases Xeronine from Proxeronine. A paper entitled, "The Pharmacologically Active Ingredient of Noni" by R. M. Heinicke of the University of Hawaii, indicates that *Morinda citrifolia* is "the best raw material to use for the isolation of xeronine," because of the building blocks of Proxeronine and Proxeronase. These building blocks aid in the isolation and production of Xeronine within the body. The function of the essential nutrient Xeronine is fourfold.

First, Xeronine serves to activate dormant enzymes found in the small intestines. These enzymes are critical to efficient digestion, calm nerves, and overall physical and emotional energy.

Second, Xeronine protects and keeps the shape and suppleness of protein molecules so that they may be able to pass through the cell walls and be used to form healthy tissue. Without these nutrients going into the cell, the cell cannot perform its job efficiently. Without Proxeronine to produce Xeronine our cells, and subsequently the body, suffer.

Third, Xeronine assists in enlarging the membrane pores of the cells. This enlargement allows for larger chains of peptides (amino acids or proteins) to be admitted into the cell. If these chains are not used they become waste.

Fourth, Xeronine, which is made from Proxeronine, assists in enlarging the pores to allow better absorption of nutrients.

Each tissue has cells, which contain proteins, which have receptor sites for the absorption of Xeronine. Certain of these proteins are the inert forms of enzymes, which require absorbed Xeronine to become active. Thus Xeronine, by converting the body's procollagenase system into a specific protease, quickly and safely removes the dead tissue from skin. Other proteins become potential receptor sites for hormones after they react with Xeronine. Thus the action of *Morinda citrifolia* in making a person feel well is probably caused by Xeronine converting certain brain receptor proteins into active sites for the absorption of the endorphin, the well being hormones. Other proteins form pores through membranes in the intestines, the blood vessels and other body organs. Absorbing Xeronine on these proteins changes the shape of the pores and thus affects the passage of molecules through the membranes.

Because of its many benefits, *Morinda citrifolia* has been known to provide a number of anecdotal effects in individuals having cancer, arthritis, headaches, indigestion, malignancies, broken bones, high blood pressure, diabetes, pain, infection, asthma, toothaches, blemishes, immune system failure, and others.

The compositions containing *Morinda citrifolia* may be in a form suitable for oral use, for example, as tablets, or lozenges, aqueous or oily suspensions, dispersible powders or granules, emulsions, syrups or elixirs. Compositions intended for oral use may be prepared according to any method known in the art for the manufacture of *Morinda citrifolia* compositions and such compositions may contain one or more agents selected from the group consisting of sweetening agents, flavoring agents, coloring agents and preserving agents. Tablets contain *Morinda citrifolia* in admixture with non-toxic pharmaceutically acceptable excipients, which are suitable for the manufacture of tablets. These excipients may be for example, inert diluents, granulating and disintegrating agents, binding agents, and lubricating agents. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Aqueous suspensions contain the *Morinda citrifolia* in admixture with excipients suitable for the manufacture of aqueous suspensions. Such excipients are suspending agents, for example, sodium carboxymethyl-cellulose, methylcellulose, hydroxy-propylmethycellulose, sodium alginate, polyvinyl-pyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethylene-oxycetanol, or condensation products of ethylene oxide with partial esters derived from fatty acids and a hexitol such as polyoxyethylene sorbitor monooleate, or condensation products of ethylene oxide with partial esters derived from fatty acids and hexitol anhydrides, for example polyethylene sorbitan monooleate.

### 2. Formulations and Methods of Administration

The present invention provides oral care methods and formulations comprising *Morinda citrifolia* such as toothpaste, gels, tooth powders, mouthwashes, mouth rinses, gums, mouth sprays and lozenges comprising *Morinda citrifolia.* The form of *Morinda citrifolia* can be juice of fruits, its extracts, fruit juice concentrate, noni oil, leaf powder, or leaf extracts. The *Morinda citrifolia* is incorporated into various carriers or nutraceutical compositions suitable for *in vivo* treatment of mammals. The processed *Morinda citrifolia* may be incorporated into a dentifrice paste or gel, powder, granules, disinteragable tablet, mouthwash, lozenges or chewing gum. The compositions may further comprise water, flavoring agents, active compounds, emulsifier, alcohol, sweeteners, thickening agents, surfactants, suspending agents, astringent and toning drug extracts, flavor correctants, abrasives or polishes, deodorizing agents, preservatives, flavoring buffers, whitening agents, wound-healing and inflammation inhibiting substances, colorants, dyes, pigments, abrasives, polishes, antimicrobial agents, pH buffers and other additives and fillers. The oral care compositions may also comprise water, flavoring agents, and other active compounds. In addition, the oral care compositions may comprise other ingredients selected from the group consisting of emulsifier, alcohol, sweeteners, thickening agents, surfactants, astringent and toning drug extracts, flavor correctants, abrasives or polishes, deodorizing agents, preservatives, flavoring buffers, whitening agents, wound-healing and inflammation inhibiting substances, colorants, dyes, pigments, abrasives, polishes, antimicrobial agents, pH buffers, and the like and combinations thereof, as well as other additives and fillers, the selection and amount of which will depend on the nature of the oral care composition.

Active ingredients may be extracted out of various parts of the *Morinda citrifolia* plants using various alcohol or alcohol-based solutions, such as methanol, ethanol, and ethyl acetate, and other alcohol-based derivatives using any known process in the art. The processed *Morinda citrifolia* product is an active ingredient or contains one or more active ingredients, such as Quercetin and Rutin, and others, for effectuating oral care. The active ingredients of Quercetin and Rutin are present in amounts by weight ranging from 0.01 - 10 percent of the total formulation or composition. These amounts may be concentrated as well into a more potent concentration in which they are present in amounts ranging from 10 to 100 percent. Additionally, chemical and mechanical methods of extraction are contemplated by the present invention including chromatography systems

The processed *Morinda citrifolia* product may be formulated with various other ingredients to produce various compositions, such as a nutraceutical composition, an internal composition, or others. The ingredients to be utilized in a nutraceutical composition are any that are safe for introduction into the body of a mammal, and particularly a human, and may exist in various forms, such as liquids, tablets, lozenges, aqueous or oily solutions, dispersible powders or granules, emulsions, syrups, elixirs, etc. Moreover, since the nutraceutical composition will most likely be consumed orally, it may contain one or more agents selected from the group consisting of sweetening agents, flavoring agents, coloring agents, preserving agents, and other medicinal agents as directed.

The carrier medium may comprise any ingredient capable of being introduced into the body of a mammal, and that is capable of providing the carrying medium to the processed *Morinda citrifolia* product. Specific carrier mediums formulations are well known in the art and are not described in detail herein. The purpose of the carrier medium is as stated, to provide a means to embody the processed *Morinda citrifolia* product into the body of the subject to be treated. The carrier for the components of the present compositions can be any orally-acceptable vehicle suitable for use in the oral cavity. Such carriers include the usual components of toothpastes, tooth powders, prophylaxis pastes, lozenges, gums and the like and are more fully described hereinafter.

### Flavoring and Sweetening Agents

Flavoring agents useful for the invention are any food grade or pharmaceutically acceptable flavoring agent, and the specific flavoring agents will depend on the type of oral care composition. Preferably, the flavoring agent comprises natural flavoring oils, including those selected from the group comprised of oil of peppermint, oil of wintergreen, oil of spearmint, clove bud oil, parsley oil, eucalyptus oil and the like. Combinations of oils and oils with other flavoring agents can also be used. Suitable flavoring agents also may be selected from a list comprised of menthol, menthane, anethole, methyl salicylate, eucalyptol, cassia, 1-methyl acetate, sage, eugenol, oxanone, alpha-irisone, marjoram, lemon, orange, propenyl guaethol acetyl, cinnamon, vanilla, thymol, linalool, cinnamaldehyde glycerol acetal and the like, and combinations thereof. The flavoring agent may comprise combinations of natural flavoring oils and other flavoring agents such as the compounds identified above. Also, the flavoring agent may comprise cooling agents such as menthol, N-substituted p-menthane-3-carboxamides (such as N-ethyl p-methane-3-carboxamide), 3,1-methoxy propane 1,2-diol and the like, or combinations thereof. Flavoring agents are generally used in the compositions at levels of from about 0.001% to about 5%, by weight of the composition.

Any food grade and/or pharmaceutically acceptable sweetener maybe used in the mouthwash, mouth rinse, mouth spray, gum or lozenge compositions, including saccharin, fructose, xylitol, saccharin salts, thaumatin, aspartame, D-tryptophan, dihydrochalcones, acesulfame and cyclamate salts, especially sodium cyclamate and sodium saccharin, and combinations thereof.

In addition to flavoring and sweetening agents, coolants, salivating agents, warming agents, and numbing agents can be used as optional ingredients in compositions of the present invention. Preferred warming agents include capsicum and nicotinate esters, such as benzyl nicotinate. Preferred numbing agents include benzocaine, lidocaine, clove bud oil, and ethanol. These agents are present in the compositions at a level of from about 0.001% to about 10%, preferably from about 0.1% to about 1%, by weight of the composition.

The coolant can be any of a wide variety of materials. Included among such materials are carboxamides, menthol, ketals, diols, and mixtures thereof. Preferred coolants in the present compositions are the paramenthan carboxyamide agents such as N-ethyl-p-menthan-3-carboxamide, known commercially as "WS-3", N,2,3-trimethyl-2-isopropylbutanamide, known as "WS-23," and mixtures thereof. Additional preferred coolants are selected from the group consisting of menthol, 3-1-menthoxypropane-1,2-diol, menthone glycerol acetal, and menthyl lactate. The terms menthol and menthyl as used herein include dextro- and levorotatory isomers of these compounds and racemic mixtures thereof. TK-10 is described in U.S. Pat. No. 4,459,425, Amano et al., issued Jul. 10, 1984. WS-3 and other agents are described in U.S. Pat. No. 4,136,163, Watson, et al., issued Jan. 23, 1979.

### Other Active Ingredients

The active compounds of the oral care composition will depend on the nature and use of the composition. In general, the active compounds for oral care compositions mask oral malodor, attack the chemicals that bring about the oral malodor, kill or inhibit growth of the bacteria in the mouth that cause breath malodor or halitosis, attack tartar, remove dirt from the teeth and mouth and/or whiten teeth. For example, in embodiments of the invention where the oral care compositions are in the form of mouthwashes, mouth rinses, gums, mouth sprays, lozenges and the like, the active components include oral hygiene actives, antibacterial substances, desensitizing agents, antiplaque agents and combinations thereof, such as those selected from the group consisting of chlorine dioxide, fluoride, alcohols, triclosan, domiphen bromide, cetyl pridinium chlorine, calcium lactate, calcium lactate salts and the like, and combinations thereof. In embodiments of the invention where the oral care compositions are in the form of dentrifices, such as toothpaste, gels, and the like, the active components include oral hygiene actives, antibacterial substances, desensitizing agents, antiplaque agents and combinations thereof, such as those selected from the group consisting of sodium fluoride, stannous fluoride, sodium monofluorophosphate, triclosan, cetyl pyridium chloride, zinc salts, pyrophosphate, calcium lactate, calcium lactate salts, 1-hydroxyethane-1,2-diphosphonic acid, 1-phosphonopropane-1,2,3-tricarboxylic acid, azacycloalkane-2,2-diphospho- nic acids, cyclic aminophosphonic acids and the like, and combinations thereof.

The present oral compositions may also include other active agents, such as antimicrobial agents. Included among such agents are water insoluble non-cationic antimicrobial agents such as halogenated diphenyl ethers, phenolic compounds including phenol and its homologs, mono and poly-alkyl and aromatic halophenols, resorcinol and its derivatives, bisphenolic compounds and halogenated salicylanilides, benzoic esters, and halogenated carbanilides. The water soluble antimicrobials include quaternary ammonium salts and bis-biquanide salts, among others. Triclosan monophosphate is an additional water soluble antimicrobial agent. The quaternary ammonium agents include those in which one or two of the substitutes on the quaternary nitrogen has a carbon chain length (typically alkyl group) from about 8 to about 20, typically from about 10 to about 18 carbon atoms while the remaining substitutes (typically alkyl or benzyl group) have a lower number of carbon atoms, such as from about 1 to about 7 carbon atoms, typically methyl or ethyl groups. Dodecyl trimethyl ammonium bromide, tetradecylpyridinium chloride, domiphen bromide, N-tetradecyl-4-ethyl pyridinium chloride, dodecyl dimethyl (2-phenoxyethyl) ammonium bromide, benzyl dimethylstearyl ammonium chloride, cetyl pyridinium chloride, quaternized 5-amino-1,3-bis(2-ethyl-hexyl)-5-methyl hexa hydropyrimidine, benzalkonium chloride, benzethonium chloride and methyl benzethonium chloride are exemplary of typical quaternary ammonium antibacterial agents. Other compounds are bis[4-(R-amino)-1-pyridinium] alkanes as disclosed in U.S. Pat. No. 4,206,215, issued Jun. 3, 1980, to Bailey. Other antimicrobials such as copper bisglycinate, copper glysinate, zinc citrate, and zinc lactate may also be included. Enzymes are another type of active that may be used in the present compositions. Useful enzymes include those that belong to the category of proteases, lytic enzymes, plaque matrix inhibitors and oxidases: Proteases include papain, pepsin, trypsin, ficin, bromelin; cell wall lytic enzymes include lysozyme; plaque matrix inhibitors include dextranses, mutanases; and oxidases include glucose oxidase, lactate oxidase, galactose oxidase, uric acid oxidase, peroxidases including horse radish peroxidase, myeloperoxidase, lactoperoxidase, chloroperoxidase. The oxidases also have whitening/cleaning activity, in addition to anti-microbial properties. Such agents are disclosed in U.S. Pat. No. 2,946,725, Jul. 26, 1960, to Norris et al. and in U.S. Pat. No. 4,051,234, Sep. 27, 1977 to Gieske et al. Other antimicrobial agents include chlorhexidine, triclosan, triclosan monophosphate, and flavor oils such as thymol. Triclosan and other agents of this type are disclosed in Parran, Jr. et al., U.S. Pat. No. 5,015,466, issued May 14, 1991, and U.S. Pat. No. 4,894,220, Jan. 16, 1990 to Nabi et al. These agents, which provide anti-plaque benefits, may be present at levels of from about 0.01 % to about 5.0%, by weight of the dentifrice composition.

### Thickening Agents

In preparing toothpaste or gels, one may add some thickening material to provide a desirable consistency of the composition, to provide desirable active release characteristics upon use, to provide shelf stability, and to provide stability of the composition, etc. Preferred thickening agents are carboxyvinyl polymers, carrageenan, hydroxyethyl cellulose, laponite and water soluble salts of cellulose ethers such as sodium carboxymethylcellulose and sodium carboxymethyl hydroxyethyl cellulose. Natural gums such as gum karaya, xanthan gum, gum arabic, and gum tragacanth can also be used. Colloidal magnesium aluminum silicate or finely divided silica can be used as part of the thickening agent to further improve texture.

The thickening agent or binder for the dentrifice, may be selected from the group consisting of finely particulate gel silicas and nonionic hydrocolloids, such as carboxmethyl cellulose, sodium hydroxymethyl cellulose, hydroxyethyl cellulose, hydroxypropyl guar, hydroxyethyl starch, polyvinyl pyrrolidone, vegetable gums, such as tragacanth, agar, carrageenans, gum arabic, xanthan gum, guar gum, locust bean gum, carboxyvinyl polymers, fumed silica, silica clays and the like and combinations thereof. The thickening agent or binder may be used with or without a carrier, such as glycerin, polyethylene glycol (PEG-400), or combinations thereof, however, when a carrier is used, up to about 5% thickening agent or binder, preferably from about 0.1% to about 1.0%, is combined with about 95.0% to about 99.9% carrier, preferably about 99.0% to about 99.9%, based on the total weight of the thickening agent/carrier combination. A preferred class of thickening or gelling agents includes a class of homopolymers of acrylic acid crosslinked with an alkyl ether of pentaerythritol or an alkyl ether of sucrose, or carbomers.

Copolymers of lactide and glycolide monomers, the copolymer having the molecular weight in the range of from about 1,000 to about 120,000 (number average), are useful for delivery of actives into the periodontal pockets or around the periodontal pockets as a "subgingival gel carrier." These polymers are described in U.S. Pat. No. 5,198,220, issued Mar. 30, 1993 and U.S. Pat. No. 5,242,910, issued Sep. 7, 1993, both to Damani, and U.S. Pat. No. 4,443,430, to Mattei, issued Apr. 17, 1984. Thickening agents in an amount from about 0.1% to about 15%, preferably from about 2% to about 10%, more preferably from about 4% to about 8%, by weight of the total toothpaste or gel composition, can be used. Higher concentrations can be used for chewing gums, lozenges (including breath mints), sachets, non-abrasive gels and subgingival gels.

Any food grade or pharmaceutically acceptable thickening agent or binder may be used in the mouthwash, mouth rinse, mouth spray, gum or lozenge compositions. The thickening agent or binder may be dispersed in a carrier, such as glycerin, polyethylene glycol or combinations thereof (thickening agent/carrier dispersion). Thickening agents and binders are those selected from the group consisting of xanthan gum, polymeric polyester compounds, natural gums (e.g. gum karaya, gum arabic, gum tragacanth), carrageenan, hydroxymethyl cellulose, methyl cellulose, carboxymethylcellulose, arrowroot powder, starches, particularly corn starch and potato starch and the like, and combinations thereof. The thickening agent or binder may be used with or without a carrier, however, when a carrier is used, up to about 5% thickening agent or binder, preferably from about 0.1% to about 1.0%, is combined with about 95.0% to about 99.9% carrier, preferably about 99.0% to about 99.9%, based on the total weight of the thickening agent/carrier combination.

### Clouding Agents

Clouding agents that may be used in the mouthwash, mouth rinse, mouth spray, gum or lozenge compositions include those selected from the group consisting of calcium citrate, esters of wood rosin, vegetable gum emulsion, caprylic/capric triglycerides, certain gums like guar gum or gum arabic and high-stability oils.

### Abrasives or Polishes

Any of the customary abrasives or polishes may be used, including those selected from the group consisting of chalk, calcium carbonate, dicalcium phosphate, insoluble sodium metaphosphate, aluminum silicates, calcium pyrophosphate, finely particulate synthetic resins, silicas, aluminum oxide, aluminum oxide trihydrate, hydroyapatite, and the like, or combinations thereof. The abrasive or polishes may, preferably be, completely or predominantly finely particulate xerogel silica, hydrogel silica, precipitated silica, aluminum oxide trihydrate and finely particulate aluminum oxide or combinations thereof.

### Surfactants

Surfactants useful in the toothpastes or gels, are those selected from the group consisting of anionic high-foam surfactants, such as linear sodium C₁₂₋₁₈ alkyl sulfates; sodium salts of C₁₂₋₁₆ linear alkyl polyglycol ether sulfates containing from 2 to 6 glycol ether groups in the molecule; alkyl-(C₁₂₋₁₆)-benzene sulfonates; linear alkane-(C₁₂₋₁₈)-sulfonates; sulfosuccinic acid mono-alkyl-(C₁₂₋₁₈)-esters; sulfated fatty acid monoglycerides; sulfated fatty acid alkanolamides; sulfoacetic acid alkyl-(C₁₂₋₁₈)-esters; and acyl sarcosides, acyl taurides and acyl isothionates all containing from 8 to 18 carbon atoms in the acyl moiety. Nonionic surfactants, such as ethoxylates of fatty acid mono- and diglycerides, fatty acid sorbitan esters and ethylene oxide-propylene oxide block polymers are also suitable. Particularly preferred surfactants are sodium lauryl sulfate and sacrosinate. Combinations of surfactants can be used.

One of the preferred optional agents of the present invention is a surfactant, preferably one selected from the group consisting of sarcosinate surfactants, isethionate surfactants and taurate surfactants. Preferred for use herein are alkali metal or ammonium salts of these surfactants. Most preferred herein are the sodium and potassium salts of the following: lauroyl sarcosinate, myristoyl sarcosinate, palmitoyl sarcosinate, stearoyl sarcosinate and oleoyl sarcosinate. This surfactant can be present in the compositions of the present invention from about 0.1% to about 2.5%, preferably from about 0.3% to about 2.5% and most preferably from about 0.5% to about 2.0% by weight of the total composition. Other suitable compatible surfactants can optionally be used or in combination with the sarcosinate surfactant in the compositions of the present invention. Suitable optional surfactants are described more fully in U.S. Pat. No. 3,959,458, May 25, 1976 to Agricola et al.; U.S. Pat. No. 3,937,807, Feb. 10, 1976 to Haefele; and U.S. Pat. No. 4,051,234, Sep. 27, 1988 to Gieske et al.

Preferred anionic surfactants useful herein include the water-soluble salts of alkyl sulfates having from 10 to 18 carbon atoms in the alkyl radical and the water-soluble salts of sulfonated monoglycerides of fatty acids having from 10 to 18 carbon atoms. Sodium lauryl sulfate and sodium coconut monoglyceride sulfonates are examples of anionic surfactants of this type. Mixtures of anionic surfactants can also be utilized.

Preferred cationic surfactants useful in the present invention can be broadly defined as derivatives of aliphatic quaternary ammonium compounds having one long alkyl chain containing from about 8 to 18 carbon atoms such as lauryl trimethylammonium chloride; cetyl pyridinium chloride; cetyl trimethylammonium bromide; di-isobutylphenoxyethyl-dimethylbenzylammonium chloride; coconut alkyltrimethylammonium nitrite; cetyl pyridinium fluoride; etc. Preferred compounds are the quaternary ammonium fluorides described in U.S. Pat. No. 3,535,421, Oct. 20, 1970, to Briner et al., where said quaternary ammonium fluorides have detergent properties. Certain cationic surfactants can also act as germicides in the compositions disclosed herein. Cationic surfactants such as chlorhexidine, although suitable for use in the current invention, are not preferred due to their capacity to stain the oral cavity's hard tissues. Persons skilled in the art are aware of this possibility and should incorporate cationic surfactants only with this limitation in mind.

Preferred nonionic surfactants that can be used in the compositions of the present invention can be broadly defined as compounds produced by the condensation of alkylene oxide groups (hydrophilic in nature) with an organic hydrophobic compound which may be aliphatic or alkylaromatic in nature. Examples of suitable nonionic surfactants include the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

Preferred zwitterionic synthetic surfactants useful in the present invention can be broadly described as derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate or phosphonate.

Preferred betaine surfactants are disclosed in U.S. Pat. No. 5,180,577 to Polefka et al., issued Jan. 19, 1993. Typical alkyl dimethyl betaines include decyl betaine or 2-(N-decyl-N,N-dimethylammonio) acetate, coco betaine or 2-(N-coc-N, N-dimethylammonio) acetate, myristyl betaine, palmityl betaine, lauryl betaine, cetyl betaine, cetyl betaine, stearyl betaine, etc. The amidobetaines are exemplified by cocoamidoethyl betaine, cocoamidopropyl betaine, lauramidopropyl betaine and the like. The betaines of choice are preferably the cocoamidopropyl betaine and, more preferably, the lauramidopropyl betaine.

### Chelating Agents

Another preferred optional agent is a chelating agent such as tartaric acid and pharmaceutically-acceptable salts thereof, citric acid and alkali metal citrates and mixtures thereof. Chelating agents are able to complex calcium found in the cell walls of the bacteria. Chelating agents can also disrupt plaque by removing calcium from the calcium bridges, which help hold this biomass intact. However, it is not desired to use a chelating agent that has an affinity for calcium that is too high, as this may result in tooth demineralization, which is contrary to the objects and intentions of the present invention.

Sodium and potassium citrate are the preferred alkali metal citrates, with sodium citrate being the most preferred. Also preferred is a citric acid/alkali metal citrate combination. Preferred herein are alkali metal salts of tartaric acid. Most preferred for use herein are disodium tartrate, dipotassium tartrate, sodium potassium tartrate, sodium hydrogen tartrate and potassium hydrogen tartrate. The amounts of chelating agent suitable for use in the present invention are about 0.1% to about 2.5%, preferably from about 0.5% to about 2.5% and more preferably from about 1.0% to about 2.5%. The tartaric acid salt chelating agent can be used alone or in combination with other optional chelating agents.

Other optional chelating agents can be used. Preferably these chelating agents have a calcium binding constant of about 10¹ to 10⁵ to provide improved cleaning with reduced plaque and calculus formation.

Another group of agents suitable for use as chelating agents in the present invention are the soluble pyrophosphates. The pyrophosphate salts used in the present compositions can be any of the alkali metal pyrophosphate salts. Specific salts include tetra alkali metal pyrophosphate, dialkali metal diacid pyrophosphate, trialkali metal monoacid pyrophosphate and mixtures thereof, wherein the alkali metals are preferably sodium or potassium. The salts are useful in both their hydrated and unhydrated forms. An effective amount of pyrophosphate salt useful in the present composition is generally enough to provide at least 1.0% pyrophosphate ion, preferably from about 1.5% to about 6%, more preferably from about 3.5% to about 6% of such ions. It is to be appreciated that the level of pyrophosphate ions is that capable of being provided to the composition (i.e., the theoretical amount at an appropriate pH) and that pyrophosphate forms other than P₂ O₇ may be present when a final product pH is established. The pyrophosphate salts are described in more detail in Kirk & Othmer, Encyclopedia of Chemical Technology, Second Edition, Volume 15, Interscience Publishers (1968).

Optional agents to be used in place of or in combination with the pyrophosphate salt include such known materials as polyamino propane sulfonic acid (AMPS), zinc citrate trihydrate, polyphosphates (e.g., tripolyphosphate; hexametaphosphate), diphosphonates (e.g., EHDP, AHP), polyphosphonates, phosphonate copolymers, polypeptides (such as polyaspartic and polyglutamic acids), and mixtures thereof. Examples of phosphonate copolymers are the diphosphonate-derivatized polymers in U.S. Pat. No. 5,011,913 to Benedict et al. A preferred polymer is diphosphonate modified polyacrylic acid. Suitable phosphonate-containing polymers such as described in U.S. Pat. No. 5,980,776 to Zakikhani, et al.

Polyphosphates are also optionally included in the present compositions. A polyphosphate is generally understood to consist of two or more phosphate molecules arranged primarily in a linear configuration, although some cyclic derivatives may be present. In addition to pyrophosphates and tripolyphosphate, which are technically polyphosphates, also desired are the polyphosphates having around four or more phosphate, i.e., tetrapolyphosphate and hexametaphosphate, among others. Polyphosphates larger than tetrapolyphosphate usually occur as amorphous glassy materials. Preferred in this invention are the linear "glassy" polyphosphates. These polyphosphates may be used alone or in a combination thereof.

Still another possible group of chelating agents suitable for use in the present invention are the anionic polymeric polycarboxylates. Such materials are well known in the art, being employed in the form of their free acids or partially or preferably fully neutralized water soluble alkali metal (e.g. potassium and preferably sodium) or ammonium salts. Preferred are 1:4 to 4:1 copolymers of maleic anhydride or acid with another polymerizable ethylenically unsaturated monomer, preferably methyl vinyl ether (methoxyethylene) having a molecular weight (M.W.) of about 30,000 to about 1,000,000.

Other operative polymeric polycarboxylates include those such as the 1:1 copolymers of maleic anhydride with ethyl acrylate, hydroxyethyl methacrylate, N-vinyl-2-pyrollidone, or ethylene, and copolymers of acrylic acid with methyl or hydroxyethyl methacrylate, methyl or ethyl acrylate, isobutyl vinyl ether or N-vinyl-2-pyrrolidone.

Additional operative polymeric polycarboxylates are disclosed in U.S. Pat. No. 4,138,477, Feb. 6, 1979 to Gaffar and U.S. Pat. No. 4,183,914, Jan. 15, 1980 to Gaffar et al. and include copolymers of maleic anhydride with styrene, isobutylene or ethyl vinyl ether, poly-acrylic, polyitaconic and polymaleic acids, and sulfoacrylic oligomers of M.W. as low as 1,000 available as Uniroyal ND-2.

### Fluoride Source

It is common to have an additional water-soluble fluoride compound present in dentifrices and other oral compositions in an amount sufficient to give a fluoride ion concentration in the composition at 25°C, and/or when it is used of from about 0.0025% to about 5.0% by weight, preferably from about 0.005% to about 2.0% by weight, to provide additional anticaries effectiveness. A wide variety of fluoride ion-yielding materials can be employed as sources of soluble fluoride in the present compositions. Examples of suitable fluoride ion-yielding materials are found in U.S. Pat. No. 3,535,421, Oct. 20, 1970 to Briner et al. and U.S. Pat. No. 3,678,154, Jul. 18, 1972 to Widder et al. Representative fluoride ion sources include stannous fluoride, sodium fluoride, potassium fluoride, sodium monofluorophosphate and many others. Stannous fluoride and sodium fluoride are particularly preferred, as well as mixtures thereof.

### Teeth Whitening Actives and Teeth Color Modifying Substances

Teeth whitening actives that may be used in the oral care compositions of the present invention include bleaching or oxidizing agents such as peroxides, perborates, percarbonates, peroxyacids, persulfates, metal chlorites, and combinations thereof. Suitable peroxide compounds include hydrogen peroxide, urea peroxide, calcium peroxide, and mixtures thereof. A preferred percarbonate is sodium percarbonate. Other suitable whitening agents include potassium, ammonium, sodium and lithium persulfates and perborate mono- and tetrahydrates, and sodium pyrophosphate peroxyhydrate. Suitable metal chlorites include calcium chlorite, barium chlorite, magnesium chlorite, lithium chlorite, sodium chlorite, and potassium chlorite. The preferred chlorite is sodium chlorite. Additional whitening actives may be hypochlorite and chlorine dioxide.

In addition to bleaching agents as teeth whitening agents, teeth color modifying substances may be considered among the oral care actives useful in the present invention. These substances are suitable for modifying the color of the teeth to satisfy the consumer. These substances comprise particles that when applied on the tooth surface modify that surface in terms of absorption and, or reflection of light. Such particles provide an appearance benefit when a film containing such particles is applied over the surfaces of a tooth or teeth.

Particles most useful in the present invention include pigments and colorants routinely used in the cosmetic arts. There are no specific limitations as to the pigment and, or colorant used in the present composition other than the limitation of the effect it has on the light source upon the teeth surfaces. Pigments and colorants include inorganic white pigments, inorganic colored pigments, pearling agents, filler powders and the like. Specific examples are selected from the group consisting of talc, mica, magnesium carbonate, calcium carbonate, magnesium silicate, aluminum magnesium silicate, silica, titanium dioxide, zinc oxide, red iron oxide, brown iron oxide, yellow iron oxide, black iron oxide, ferric ammonium ferrocyanide, manganese violet, ultramarine, nylon powder, polyethylene powder, methacrylate powder, polystyrene powder, silk powder, crystalline cellulose, starch, titanated mica, iron oxide titanated mica, bismuth oxychloride, and mixtures thereof. Most preferred are those selected from the group consisting of titanium dioxide, bismuth oxychloride, zinc oxide and mixtures thereof. Pigments that are generally recognized as safe, and are listed in C.T.F.A. Cosmetic Ingredient Handbook, 3rd Ed., Cosmetic and Fragrance Assn., Inc., Washington D.C. (1982).

The pigments are typically used as opacifiers and colorants. These pigments can be used as treated particles, or as the raw pigments themselves. Typical pigment levels are selected for the particular impact that is desired by the consumer. For example, for teeth that are particularly dark or stained one would typically use pigments in sufficient amount to lighten the teeth. On the other hand, where individual teeth or spots on the teeth are lighter than other teeth, pigments to darken the teeth may be useful. The levels of pigments and colorants are generally used in the range of about 0.05% to about 20%, preferably from about 0.10% to about 15% and most preferably from about 0.25% to about 10% of the composition.

### Humectants

Another optional component of the topical, oral carriers of the compositions of the subject invention is a humectant. The humectant serves to keep toothpaste compositions from hardening upon exposure to air, to give compositions a moist feel to the mouth, and, for particular humectants, to impart desirable sweetness of flavor to toothpaste compositions. The humectant, on a pure humectant basis, generally comprises from about 0% to about 70%, preferably from about 5% to about 25%, by weight of the compositions herein. Suitable humectants for use in compositions of the subject invention include edible polyhydric alcohols such as glycerin, sorbitol, xylitol, butylene glycol, polyethylene glycol, and propylene glycol, especially sorbitol and glycerin.

### Alkali Metal Bicarbonate Salt

The present invention may also include an alkali metal bicarbonate salt. Alkali metal bicarbonate salts are soluble in water and unless stabilized, tend to release carbon dioxide in an aqueous system. Sodium bicarbonate, also known as baking soda, is the preferred alkali metal bicarbonate salt. The present composition may contain from about 0.5% to about 30%, preferably from about 0.5% to about 15%, and most preferably from about 0.5% to about 5% of an alkali metal bicarbonate salt.

### Miscellaneous Carriers

Water employed in the preparation of commercially suitable oral compositions should preferably be of low ion content and free of organic impurities. Water generally comprises from about 10% to about 50%, and preferably from about 20% to about 40%, by weight of the aqueous toothpaste compositions herein. These amounts of water include the free water which is added plus that which is introduced with other materials, such as with sorbitol.

Titanium dioxide may also be added to the present composition. Titanium dioxide is a white powder, which adds opacity to the compositions. Titanium dioxide generally comprises from about 0.25% to about 5% by weight of the dentifrice compositions.

Other optional agents that may be used in the present compositions include dimethicone copolyols selected from alkyl- and alkoxy-dimethicone copolyols, such as C12 to C20 alkyl dimethicone copolyols and mixtures thereof. Highly preferred is cetyl dimethicone copolyol marketed under the Trade Name Abil EM90. The dimethicone copolyol is generally present in a level of from about 0.01% to about 25%, preferably from about 0.1% to about 5%, more preferably from about 0.5% to about 1.5% by weight. The dimethicone copolyols aid in providing positive tooth feel benefits.

### Preservatives and pH buffers

Preservatives and antimicrobial agents that may be used in the toothpaste or gels include those selected from the group consisting of p-hydroxybenzoic acid; methyl, ethyl or propyl ester; sodium sorbate; sodium benzoate, bromochlorophene, phenyl salicylic acid esters, thymol, and the like; and combinations thereof.

The pH of the present compositions is preferably adjusted through the use of buffering agents. Buffering agents, as used herein, refer to agents that can be used to adjust the pH of the compositions to a range of about 4.5 to about 9.5. Buffering agents include monosodium phosphate, trisodium phosphate, sodium hydroxide, sodium carbonate, sodium acid pyrophosphate, citric acid, and sodium citrate. Buffering agents can be administered at a level of from about 0.5% to about 10%, by weight of the present compositions. The pH of dentifrice compositions is measured from a 3:1 aqueous slurry of dentifrice, e.g., 3 parts water to 1 part toothpaste. Suitable pH buffers include those selected from the group consisting of primary, secondary or tertiary alkali phosphates, citric acid, sodium citrate, and the like or combinations thereof. Wound healing and inflammation inhibiting substances include those selected from the group consisting of allantoin, urea, azulene, camomile active substances and acetyl salicylic acid derivatives, and the like, or combinations thereof.

### Colorants

Food grade and/or pharmaceutically acceptable coloring agents, or colorants, as would be understood to one skilled in the art, can be used in the present invention. An example of a pigment is titanium dioxide (such as U.S.P. grade available from Whittaker, Clark & Daniels) to provide a bright white color. Food grade and/or pharmaceutically acceptable coloring agents, dyes, or colorants, as would be understood to one skilled in the art, can be used in these compositions, including FD&C colorants including primary FD&C Blue No. 1, FD&C Blue No. 2, FD&C Green No. 3, FD&C Yellow No. 5, FD&C Yellow No. 6, FD&C Red No. 3, FD&C Red No. 33 and FD&C Red No. 40 and lakes FD&C Blue No. 1, FD&C Blue No. 2, FD&C Yellow No. 5, FD&C Yellow No. 6, FD&C Red No. 2, FD&C Red No. 3, FD&C Red No. 33, FD&C Red No. 40 and combinations thereof. Like colorants and dyes may also be used. A composition preferably contains from about 0.1 % to about 10% of these agents, preferably from about 0.1% to about 1%, by weight of the composition.

Typical mouthwash, mouth rinse, mouth spray, gum and lozenge compositions will comprise about 30% to about 80% water, about 2% to about 35% humectant, about 1% to about 50% active compounds comprising at least *Morinda citrfolia*, about 0.01 % to about 0.50% of at least one sweetener, about 0.01 % to about 0.50% of at least one thickening agent or binder which may be dispersed in about 2.5% to about 10% of a carrier, such as glycerin, polyethylene glycol (PEG-400) or combinations thereof, about 0.03% to about 3% of at least one surfactant and about 0.01% to about 1% of at least one flavoring agent. Optionally, the typical mouthwash, mouth rinse, mouth spray, gum or lozenge compositions can comprise about 0.01 % to about 1.0% colorants, which includes dyes and pigments and about 0.01 % to about 1.0% clouding agents. The compositions may further comprise about 0.01% to about 1.0% titanium dioxide.

In another embodiment of the invention, the oral care composition is in the form of a dentifrice, such as toothpaste or gels. Toothpaste and gels are generally understood to be paste-like or gel-like preparations that are applied directly to the teeth generally by brushing, and dentifrices may be a combination of pastes and gels, as well as combinations of gels or toothpaste with mouthwashes or mouth rinses. Gums and lozenges may also be used as dentifrices provided that these include the active ingredients normally associated with dentifrice compositions.

The dentrifice composition will generally comprise from about 5% to about 20% water, about 5% to about 75% humectant, about 0.25% to about 3.0% of at least one thickening agent or binder which may be dispersed in about 2.5% to about 10% of a carrier, such as glycerin, polyethylene glycol (PEG-400), or combinations thereof, about 0.01% to about 0.05% sweeteners, about 5% to about 40% abrasives and polishes, about 0.5% to about 3.0% surfactants, about 0.01 % to about 50.0% active compounds comprising *Morinda citrifolia* and which may include oral hygiene actives, antibacterial substances, desensitizing agents, antiplaque agents and combinations thereof, and about 0.25% to about 3.0% flavoring agents. The dentrifice compositions may also comprise fillers and additives, such as about 0.05% to about 1.0% preservative and/or antimicrobial agents, about 0.50% to about 10.0% buffers, about 0.05% to about 5.0% wound healing and inflammation-inhibiting substances, about 0.01% to about 2.0% colorants, such as colors, dyes or particles for special effects, and from about 0.05% to about 10.0% whitening agents, such as hydrogen peroxide and pyrophosphates.

Several embodiment of formulations are provided below. However, these are only intended to be exemplary as one ordinarily skilled in the art will recognize other formulations or compositions comprising the processed *Morinda citrifolia* product.

In one exemplary embodiment, the present invention further features a method of administering a nutraceutical composition comprising *Morinda citrifolia* for oral care. The method comprises the steps of (a) formulating a nutraceutical composition comprising in part a processed *Morinda citrifolia* product present in an amount between about 0.01 and 95 percent by weight, wherein the composition also comprises a carrier, such as water or purified water, and other natural or artificial ingredients; (b) administering the nutraceutical composition into the body such that the processed *Morinda citrifolia* product is sufficiently internalized; (c) repeating the above steps as often as necessary to provide an effective amount of the processed *Morinda citrifolia* product.

The step of administering the nutraceutical composition into the body comprises ingesting the composition orally through one of several means. Specifically, the nutraceutical composition may be formulated as a liquid, gel, solid, or some other type that would allow the composition to be quickly and conveniently digested, masticated or otherwise exposed to the tissues present in a mammal's mouth. Further the processed *Morinda citrifolia* may be incorporated into a dentifrice paste or gel, powder, granules, disinteragable tablet, mouthwash, lozenges or chewing gum.

The following tables illustrate or represent some of the preferred formulations or compositions contemplated by the present invention. As stated, these are only intended as exemplary embodiments and are not to be construed as limiting in any way.

**TABLE 1: Dentifrice Powder, Granules, or Disintegrable Tablets**

| Ingredient | Weight % |
|---|---|
| calcium carbonate | 50.0 |
| | |
| *Morinda citrifolia* | 31.0 |
| microcrystalline cellulose | 14.6 |
| PluronieF121 | 2.0 |
| xanthan gum | 1.0 |
| Methocel K15MP | 0.5 |
| flavor | 0.5 |
| monoantmonium | 0.4 |
| gtyeyrrhizinate | |

One of the embodiments of this invention is a dentifrice in paste form. A typical formula for a dentifrice paste according to the claims of this invention follows in table two.

**TABLE 2: Dentifrice Paste**

| Ingredient | Weight % |
|---|---|
| *Morinda citrifolia* | 35.9 |
| Water | 33.4 |
| Calcium carbonate | 24.1 |
| | |
| Pluronic | 4.0 |
| Cellulose gum | 1.5 |
| | |
| Methocel | 0.5 |
| Dipotassium glycyrrhizinate | 0.4 |
| One of the embodiments of this invention is a dentifrice in gel form. A typical formula for | |
| Flavor | 0.2 |

**TABLE 3: Dentifrice Gel**

| Ingredient | Weight % |
|---|---|
| *Morinda citrifolia* | 38.0 |
| Water | 33.3 |
| thickening silica | 10 |
| | |
| Glycerin | 7.3 |
| abrasive silica | 5 |
| | |
| Pluronic | 4 |
| cellulose gunm | 1.5 |
| | |
| Methocel | 0.5 |
| | |
| Disodium glycyrrhizinate | 0.2 |
| | |
| Flavor | 0.2 |

One of the embodiments of this invention is a plaque preventing hard or soft lozenge to be sucked on by the user. A typical formula for a lozenge according to the claims of this invention is depicted in table four.

**TABLE 4: Dentifrice Lozenge**

| Ingredient | Weight % |
|---|---|
| *Morinda citrifolia* | 91.6 |
| Pluronic | 4.0 |
| cellulose gum | 1.0 |
| | |
| Methocel | 0.5 |
| calcium carbonate | 2.0 |
| Flavor | 0.5 |
| Dipotassium glycyrrhizinate | 0.4 |

One of the embodiments of this invention is a plaque preventing chewing gum. A typical formula for a chewing gum according to the claims of this invention is depicted in table five.

**TABLE 5: Dentifrice Chewing Gum**

| Ingredient | Weight % |
|---|---|
| gum base | 20.0 |
| *Morinda citrfolia* | 67.5 |
| calcium carbonale | 5.0 |
| | |
| Glycerin | 3.0 |
| Pluronic | 2.0 |
| | |
| cellulose gum | 1.0 |
| | |
| Methocel | 0.5 |
| Flavor | 0.5 |
| Monoammonium glycyrrhizinate | 0.4 |
| | |
| xanthan gum | 0.1 |

One of the embodiments of this invention is a plaque preventing mouthwash. A typical formula for a mouthwash is depicted in table six.

**TABLE 6: Mouthwash**

| Ingredient | Weight % |
|---|---|
| Water | 65.49 |
| *Morinda citrifolia* | 32.1 |
| Pluronic | 1.0 |
| Celulose gum | 0.24 |
| | |
| Methocel | 0.12 |
| Flavor | 0.5 |
| Disodium glycyrrhizinate | 0.4 |
| | |
| preservative | 0.1 |
| sodium fluoride | 0.05 |

In one preferred method, a person wanting to promote oral care as described above takes, or is administered, at least one ounce of the mouth wash (depicted in table six) in the morning, and at least one ounce at night, just prior to retiring to bed. In one example, which is not meant to be limiting in any way, the beneficial *Morinda Citrifolia* is processed into Tahitian Noni^{®} juice manufactured by Morinda, Incorporated of Orem, Utah.

In another preferred method of the present invention, a person wanting to promote oral care as described above utilizes the above described dental paste with a brushing mechanism to apply the paste to the their teeth. After brushing with the dental paste of the present invention, the person holds at least one ounce of the mouth wash, of the present invention, in their mouth, for a sufficient period of time. In a preferred embodiment of the present invention this method is repeated multiple times during the day comprising early in the morning, just prior to retiring to bed and after meals. These particular methods of introducing a composition may comprise any method of actually introducing the composition to the subject for the purpose of promoting oral care. Although the particular methods are many, the present invention recognizes that the composition may be introduced by a variety of mechanisms including orally. No matter what method is employed, it is important to regulate the amount of active ingredient that the subject is exposed to so that the appropriate oral care objectives are accomplished. The following examples set forth and present the effects of oral care with *Morinda citrifolia.* These examples are not intended to be limiting in any way, but are merely illustrative of the benefits and advantages of utilizing *Morinda citrifolia* for oral care.

### 3. Promoting Oral Care

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes that come within the meaning and range of equivalency of the claims are to be embraced within their scope.

The present invention capitalizes on these properties by incorporating components of the *Morinda citrifolia* plant as constituents of oral care compositions to treat and prevent various oral and dental-related disorders. Certain embodiments of the present invention comprise an excipient base component and an active component comprising *Morinda citrifolia* in an amount up to fifty percent by weight.

### Example 1: Prevent Gum Disease

In view of the foregoing, it is an object of certain embodiments of the present invention to provide an oral dental formulation capable of boosting immune response to gum diseases or tooth decay. In this embodiment of the present invention a person wanting to promote oral care as described above utilizes the above described dentifrice paste with a brushing mechanism to apply the paste to the their teeth. After brushing with the dentifrice paste of the present invention, the person holds at least one ounce of the above described mouth wash in their mouth, for a sufficient period of time to inhibit gum diseases or tooth decay. In a preferred embodiment of the present invention this method is repeated multiple times during the day comprising early in the morning, just prior to retiring to bed and after meals. These particular methods of introducing a composition may comprise any method of actually introducing the composition to the subject for the purpose of promoting oral care including the use of the above described dentifrice powder, granules, disintegrable tablets, paste, gel, lozenge, gum or mouthwash alone or in combination with each other. No matter what method is employed, it is important to regulate the amount of active ingredient that the subject is exposed to so that the appropriate oral care objectives are accomplished.

### Example 2: Increase Collage Density

It is a further object of certain embodiments of the present invention to provide an oral dental formulation and method of reducing gingival bleeding by reducing inflammation in the gingival sulcus and increasing gingival collagen density. In this embodiment of the present invention a person wanting to promote oral care as described above utilizes the above described dentifrice paste with a brushing mechanism to apply the paste to the their teeth. After brushing with the dentifrice paste of the present invention, the person holds at least one ounce of the above described mouthwash in their mouth, for a sufficient period of time to increase gingival collagen density. In a preferred embodiment of the present invention this method is repeated multiple times during the day comprising early in the morning, just prior to retiring to bed and after meals. These particular methods of introducing a composition may comprise any method of actually introducing the composition to the subject for the purpose of promoting oral care including the use of the above described dentifrice powder, granules, disintegrable tablets, paste, gel, lozenge, gum or mouthwash alone or in combination with each other. No matter what method is employed, it is important to regulate the amount of active ingredient that the subject is exposed to so that the appropriate oral care objectives are accomplished.

### Example 3: Reduce Plaque

It is another object of certain embodiments of the present invention to provide an oral dental formulation and method of reducing periodontal attachment loss by reducing plaque formation and/or plaque adhesion to teeth and surrounding oral tissues. In this embodiment of the present invention a person wanting to promote oral care as described above utilizes the above described dentifrice paste with a brushing mechanism to apply the paste to the their teeth. After brushing with the dentifrice paste of the present invention, the person holds at least one ounce of the above described mouthwash in their mouth, for a sufficient period of time to reduce plaque formation and/or plaque adhesion to teeth and surrounding oral tissues. In a preferred embodiment of the present invention this method is repeated multiple times during the day comprising early in the morning, just prior to retiring to bed and after meals. These particular methods of introducing a composition may comprise any method of actually introducing the composition to the subject for the purpose of promoting oral care including the use of the above described dentifrice powder, granules, disintegrable tablets, paste, gel, lozenge, gum or mouthwash alone or in combination with each other. No matter what method is employed, it is important to regulate the amount of active ingredient that the subject is exposed to so that the appropriate oral care objectives are accomplished.

### Example 4: Reduce Bacterial Activity

It is another object of certain embodiments of the present invention to provide an oral dental formulation and method to reduce epithelial cell wall permeability to bacterial toxins, and further to reduce collagenase enzyme activity in the presence of bacteria. In this embodiment of the present invention a person wanting to promote oral care as described above utilizes the above described dentifrice paste with a brushing mechanism to apply the paste to the their teeth. After brushing with the dentifrice paste of the present invention, the person holds at least one ounce of the above described mouthwash in their mouth, for a sufficient period of time to reduce collagenase enzyme activity in the presence of bacteria. In a preferred embodiment of the present invention this method is repeated multiple times during the day comprising early in the morning, just prior to retiring to bed and after meals. These particular methods of introducing a composition may comprise any method of actually introducing the composition to the subject for the purpose of promoting oral care including the use of the above described dentifrice powder, granules, disintegrable tablets, paste, gel, lozenge, gum or mouthwash alone or in combination with each other. No matter what method is employed, it is important to regulate the amount of active ingredient that the subject is exposed to so that the appropriate oral care objectives are accomplished.

### Example 5: Increase Cellular Respiration

It is yet another object of certain embodiments of the present invention to provide an oral dental formulation and method to increase cellular respiration and oxygen saturation of cells in the mouth. In this embodiment of the present invention a person wanting to promote oral care as described above utilizes the above described dentifrice paste with a brushing mechanism to apply the paste to the their teeth. After brushing with the dentifrice paste of the present invention, the person holds at least one ounce of the above described mouthwash in their mouth, for a sufficient period of time to increase cellular respiration and oxygen saturation of cells in the mouth. In a preferred embodiment of the present invention this method is repeated multiple times during the day comprising early in the morning, just prior to retiring to bed and after meals. These particular methods of introducing a composition may comprise any method of actually introducing the composition to the subject for the purpose of promoting oral care including the use of the above described dentifrice powder, granules, disintegrable tablets, paste, gel, lozenge, gum or mouthwash alone or in combination with each other. No matter what method is employed, it is important to regulate the amount of active ingredient that the subject is exposed to so that the appropriate oral care objectives are accomplished.

### Example 6: Inhibit Apthus Ulcers

It is a further object of certain embodiments of the present invention to provide an oral dental formulation and method to reduce the incidence and duration of apthus ulcers in the mouth. In this embodiment of the present invention a person wanting to promote oral care as described above utilizes the above described dentifrice paste with a brushing mechanism to apply the paste to the their teeth. After brushing with the dentifrice paste of the present invention, the person holds at least one ounce of the above described mouthwash in their mouth, for a sufficient period of time to reduce the incidence and duration of apthus ulcers in the mouth. In a preferred embodiment of the present invention this method is repeated multiple times during the day comprising early in the morning, just prior to retiring to bed and after meals. These particular methods of introducing a composition may comprise any method of actually introducing the composition to the subject for the purpose of promoting oral care including the use of the above described dentifrice powder, granules, disintegrable tablets, paste, gel, lozenge, gum or mouthwash alone or in combination with each other. No matter what method is employed, it is important to regulate the amount of active ingredient that the subject is exposed to so that the appropriate oral care objectives are accomplished.

### Example 7: Remove Staining

It is another object of certain embodiments of the present invention to provide an oral dental formulation and method to clean teeth, remove surface staining from teeth, and whiten teeth. In this embodiment of the present invention a person wanting to promote oral care as described above utilizes the above described dentifrice paste with a brushing mechanism to apply the paste to the their teeth. After brushing with the dentifrice paste of the present invention, the person holds at least one ounce of the above described mouthwash in their mouth, for a sufficient period of time to clean teeth, remove surface staining from teeth, and whiten teeth. In a preferred embodiment of the present invention this method is repeated multiple times during the day comprising early in the morning, just prior to retiring to bed and after meals. These particular methods of introducing a composition may comprise any method of actually introducing the composition to the subject for the purpose of promoting oral care including the use of the above described dentifrice powder, granules, disintegrable tablets, paste, gel, lozenge, gum or mouthwash alone or in combination with each other. No matter what method is employed, it is important to regulate the amount of active ingredient that the subject is exposed to so that the appropriate oral care objectives are accomplished.

### Example 8: Enhance Dental Health

It is yet another object of certain embodiments of the present invention to provide an oral dental formulation and method to treat and prevent gum diseases and tooth decay in mammals and further to generally enhance overall dental health. In this embodiment of the present invention a person wanting to promote oral care as described above utilizes the above described dentifrice paste with a brushing mechanism to apply the paste to the their teeth. After brushing with the dentifrice paste of the present invention, the person holds at least one ounce of the above described mouthwash in their mouth, for a sufficient period of time to treat and prevent gum diseases and tooth decay in mammals and further to generally enhance overall dental health. In a preferred embodiment of the present invention this method is repeated multiple times during the day comprising early in the morning, just prior to retiring to bed and after meals. These particular methods of introducing a composition may comprise any method of actually introducing the composition to the subject for the purpose of promoting oral care including the use of the above described dentifrice powder, granules, disintegrable tablets, paste, gel, lozenge, gum or mouthwash alone or in combination with each other. No matter what method is employed, it is important to regulate the amount of active ingredient that the subject is exposed to so that the appropriate oral care objectives are accomplished.

### Example 9: Reduce Halitosis

It is a further object of certain embodiments of the present invention to provide an oral dental formulation and method to reduce halitosis in mammals. In this embodiment of the present invention a person wanting to promote oral care as described above utilizes the above described dentifrice paste with a brushing mechanism to apply the paste to the their teeth. After brushing with the dentifrice paste of the present invention, the person holds at least one ounce of the above described mouthwash in their mouth, for a sufficient period of time to reduce halitosis in mammals. In a preferred embodiment of the present invention this method is repeated multiple times during the day comprising early in the morning, just prior to retiring to bed and after meals. These particular methods of introducing a composition may comprise any method of actually introducing the composition to the subject for the purpose of promoting oral care including the use of the above described dentifrice powder, granules, disintegrable tablets, paste, gel, lozenge, gum or mouthwash alone or in combination with each other. No matter what method is employed, it is important to regulate the amount of active ingredient that the subject is exposed to so that the appropriate oral care objectives are accomplished.

### Example 10: Antimicrobal Properties of Processed Morinda Citrfolia Products

Research performed in support of this invention demonstrates that processed Morinda citrifolia Products have antimicrobial activity. The minimum inhibitory concentration (MIC) of an antibacterial is defined as the maximum dilution of the product that will still inhibit the growth of a test microorganism. The minimum lethal concentration (M LC) of an antibacterial is defined as the maximum dilution of the product that will kill a test organism. MIC/MLC values can be determined by a number of standard test procedures. The most commonly employed methods are the tube dilution method and agar dilution methods, The tube dilution method was proposed for this product to determine the MIC, and plating aliquots from dilutions demonstrating possible inhibition of growth to determine the MLC. Serial dilutions were made of the products in bacterial growth media. The test organisms were added to the dilutions of the products, incubated, and scored for growth. All tests were performed in triplicate.

This procedure is a standard assay for antimicrobials. The procedure incorporates the content and intent of the American Society for Microbiology (ASM) recommended methodology. The tube dilution method employs dilutions of the test product in a bacterial growth media, inoculation with a predetermined test organism concentration, and visualization of growth after incubation. Tube dilution procedures are limited to products, which do not precipitate or cloud the growth media within the expected endpoint range.
Cell cultures were prepared from stock, the test organisms were transferred to soybean casein digest broth (SCOB) and incubated at 37 ± 2°C for 24-48 hours for bacteria, and 20-25°C for yeast. If needed, the suspensions were adjusted to approximately 108 colony forming units (CFU) per mL, by visual turbidity, in physiological saline solution (PHSS) and a standard plate count was performed to determine starting titers. The yeast culture was plated onto Sabouraud dextrose agar (SDEX) and incubated at 20-25°C for 2-4 days, *S. mutans* was incubated at 37 ± 2°C for 3-5 days, and all other bacteria were incubated at 37 ± 2°C for 18-24 hours.

Tests were performed at a neutral pH. The pH was recorded before and after adjustments had been made. Each test product was diluted 1:2 serially in sterile water. Dilutions were selected that would show the MIC/MLC endpoint. Each test product evaluation was performed in triplicate for each organism. The product dilutions were added to an equal volume of 2X SCDB to provide an additional 1:2 dilution.

Three positive control tubes were prepared for each test organism by mixing sterile water with equal volumes of 2X SCDB. Three negative control tubes were prepared by mixing the highest dilution tested of the test product with equal volumes of 2X SCDB. No test organisms were added to these tubes. Three media control tubes were prepared by mixing sterile water with equal volumes of 2X SCDB. No test organisms were added to these tubes.
Approximately 0.05 mL of each test organism suspension was added to the sample and positive control tubes. The bacteria test tubes were incubated at 37 ± 2°C for 18-24 hours and yeast test tubes were incubated at 20-25°C for 2-4 days. After incubation, growth was scored as negative (0) or positive (+) for each tube.

Only tubes suspected of not having any growth were tested for MLC. A 1.0 mL aliquot was removed from each tube and serial 1/10 dilutions were made in neutralizer broth up to 1/1000. An aliquot of each dilution was plated on neutralizer agar(NUAG). For a positive control, 10-100 CFU were plated onto NUAG. A negative control was made by plating 2X SCDB onto NUAG. The plates were incubated 20-25°C for 2-4 days for yeast, and 37 ± 2°C for 18-24 hours for all bacteria except for *S. mutans.*

The lowest dilution of the test product tested for MLC was tested for neutralization recovery for each test organism. In triplicate, 0.5 mL aliquots of the most concentrated test product were plated on N UAG. The plates were spiked with 10-100 CFU of each test organism. For comparison, three plates of NUAG without the test product were also spiked with the same 10-100 CFU for each of the test organisms.

With the exception of *S. mutans*, all organisms were inhibited by neutralized *Morinda citrifolia* concentrate at a 1:2 concentration. None of the dilutions tested were able to demonstrate lethality for any of the organisms. Neither inhibition nor lethality was demonstrated by the neutralized Noni concentrate when tested against *S. mutans.* The MIC results for all organisms are summarized in Tables 1-7. The MLC results for each organism are summarized iii Tables 8-13. Since *S. mutaris* did not have any dilutions that were scored as having no growth for the MIC portion of the test, MLC was not performed for this organism.

The neutralization recoveries for all test organisms ranged from 40-97%. The neutralization recovery of the neutralizing media used in the study is summarized in Table twenty.

**TABLE 7: MIC Results**

| *Escherichia coil* 0157H7 ATCC #43885 | | | |
|---|---|---|---|
| DILUTION | GROWTH +/0 | | |
| 1:2 | 0 | 0 | 0 |
| 1:4 | + | + | + |
| 1:8 | + | + | + |
| 1:16 | + | + | + |
| 1:32 | + | + | + |
| 1:64 | + | + | + |
| Positive | + | + | + |
| Negative | 0 | 0 | 0 |
| Media | 0 | 0 | 0 |

| | | | |
|---|---|---|---|
| Titer: 7.0 x 10⁸CFU/mL | | | |

**TABLE 8: MIC Results**

| *Staphylococcus aureus* ATCC #6538 | | | |
|---|---|---|---|
| DILUTION | GROWTH +/0 | | |
| 1:2 | 0 | 0 | 0 |
| 1:4 | + | + | + |
| 1:8 | + | + | + |
| 1:16 | + | + | + |
| 1:32 | + | + | + |
| 1:64 | + | + | + |
| Positive | + | + | + |
| Negative | 0 | 0 | 0 |
| Media | 0 | 0 | 0 |

| | | | |
|---|---|---|---|
| Titer: 6.5 x 10⁸ CFU/mL | | | |

**TABLE 9: MIC Results**

| *Bacillus subtilis* ATCC #19659 | | | |
|---|---|---|---|
| DILUTION | GROWTH +/0 | | |
| 1:2 | 0 | 0 | 0 |
| 1:4 | + | + | + |
| 1:8 | + | + | + |
| 1:16 | + | + | + |
| 1:32 | + | + | + |
| 1:64 | + | + | + |
| Positive | + | + | + |
| Negative | 0 | 0 | 0 |
| Media | 0 | 0 | 0 |

| | | | |
|---|---|---|---|
| Titer: 8.5 x 10⁷ CFU/mL | | | |

**TABLE 10: MIC Results**

| *Salmonella choleraesuis* serotype *enteritidis* ATCC #13706 | | | |
|---|---|---|---|
| DILUTION | GROWTH +/0 | | |
| 1:2 | 0 | 0 | 0 |
| 1:4 | + | + | + |
| 1:8 | + | + | + |
| 1:16 | + | + | + |
| 1:32 | + | + | + |
| 1:64 | + | + | + |
| Positive | + | + | + |
| Negative | 0 | 0 | 0 |
| Media | 0 | 0 | 0 |

| | | | |
|---|---|---|---|
| Titer: 4.8 x 10⁸ CFU/mL | | | |

**TABLE 11: MIC Results**

| *Listeria monocytogenes* ATCC #19111 | | | |
|---|---|---|---|
| DILUTION | GROWTH +./0 | | |
| 1:2 | 0 | 0 | 0 |
| IA | + | + | + |
| 1:8 | + | + | + |
| 1:16 | + | + | + |
| 1:32 | + | + | + |
| 1:64 | + | + | + |
| Positive | + | + | + |
| Negative | 0 | 0 | 0 |
| Media | 0 | 0 | 0 |

| | | | |
|---|---|---|---|
| Titer: 3.9 x 10⁸ CFU/mL | | | |

**TABLE 12: MIC Results**

| *Candida albicans* ATCC #1 0231 | | | |
|---|---|---|---|
| DILUTION | GROWTH +/0 | | |
| 1:2 | 0 | 0 | 0 |
| 1:4 | + | + | + |
| 1:8 | + | + | + |
| 1:16 | + | + | + |
| 1:32 | + | + | + |
| 1:64 | + | + | + |
| Positive | + | + | + |
| Negative | 0 | 0 | 0 |
| Media | 0 | 0 | 0 |

| | | | |
|---|---|---|---|
| Titer 1.3 x 10⁸ CFU/mL | | | |

**TABLE 13: MIC Results**

| *Streptococcus mutans* ATCC #25175 | | | |
|---|---|---|---|
| DILUTION | GROWTH +/0 | | |
| 1:2 | + | + | + |
| 1:4 | + | + | + |
| 1:8 | + | + | + |
| Positive | + | + | + |
| Negative | 0 | 0 | 0 |
| Media | 0 | 0 | 0 |

| | | | |
|---|---|---|---|
| Titer: 1.0 x 10⁸ CFU/mL | | | |

**TABLE 14: MLC Plate Counts**

| *Escherichia coli* 01 57H7 ATCC #43 888 | | | | | |
|---|---|---|---|---|---|
| DILUTION | REPLICATE | DILUTION | | | |
| | | 10° | 10⁻¹ | 10⁻² | 10⁻³ |
| 1:2 | 1 | TNTC | TNTC | TNTC | 245 |
| | 2 | TNTC | TNTC | TNTC | 239 |
| | 3 | TNTC | TNTC | TNTC | 215 |

| | | | | | |
|---|---|---|---|---|---|
| Note: Volume plated = 0.5 mL TNTC = Too Numerous To Count. | | | | | |

**TABLE 15: MLC Plate Counts**

| *Staphylococcus aureus* ATCC #6538 | | | | | |
|---|---|---|---|---|---|
| DILUTION | REPLICATE | DILUTION | | | |
| | | 10⁰ | 10⁻¹ | 10² | 10³ |
| 1:2 | 1 | TNTC | TNTC | TNTC | 200 |
| | 2 | TNTC | TNTC | TNTC | 134 |
| | 3 | TNTC | TNTC | TNTC | 114 |

| | | | | | |
|---|---|---|---|---|---|
| TNTC = Too Numerous To Count. | | | | | |

**TABLE 16: MLC Plate Counts**

| *Bacillus subtilis* ATCC #19659 | | | | | |
|---|---|---|---|---|---|
| DILUTION | REPLICATE | DILUTION | | | |
| | | 10⁰ | 10⁻¹ | 10² | 10³ |
| 1:2 | 1 | 27 | 3 | 0 | 0 |
| | 2 | 25 | 2 | 0 | 0 |
| | 3 | 18 | 2 | 0 | 0 |

**TABLE 17: MLC Plate Counts**

| *Salmonella choleraesuis* serotype *enteritidis* ATCC #13706 | | | | | |
|---|---|---|---|---|---|
| DILUTION | REPLICATE | DILUTION | | | |
| | | 10⁰ | 10⁻¹ | 10² | 10³ |
| 1:2 | 1 | TNTC | TNTC | 41 | 7 |
| | 2 | TNTC | TNTC | 75 | 5 |
| | 3 | TNTC | TNTC | 63 | 6 |

| | | | | | |
|---|---|---|---|---|---|
| TNTC = Too Numerous To Count. | | | | | |

**TABLE 18: MLC Plate Counts**

| *Listeria moriocytogenes* ATCC #19111 | | | | | |
|---|---|---|---|---|---|
| DILUTION | REPLICATE | DILUTION | | | |
| | | 10⁰ | 10⁻¹ | 10² | 10³ |
| 1:2 | 1 | TNTC | TNTC | TNTC | 109 |
| | 2 | TNTC | TNTC | TNTC | 109 |
| | 3 | TNTC | TNTC | TNTC | 179 |

| | | | | | |
|---|---|---|---|---|---|
| TNTC = Too Numerous To Count. | | | | | |

**TABLE 19: MLC Plate Counts**

| *Candid albicans* ATCC #1 0231 | | | | | |
|---|---|---|---|---|---|
| DILUTION | REPLICATE | DILUTION | | | |
| | | 10⁰ | 10⁻¹ | 10² | 10³ |
| 1:2 | 1 | INTO | TNTC | TNTC | 168 |
| | 2 | TNTC | TNTC | TNTC | 117 |
| | 3 | TNTC | TNTC | TNTC | 13 |

| | | | | | |
|---|---|---|---|---|---|
| TNTC = Too Numerous To Count. | | | | | |

**TABLE 20: Neutralization**

| ORGANISM | POSITIVE COUNT | | | | NEUTRALIZATION COUNT | | | | PERCENT RECOVERY |
|---|---|---|---|---|---|---|---|---|---|
| | 1 | 2 | 3 | AVE | 1 | 2 | 3 | AVE | |
| E.coli | 60 | 63 | 58 | 60 | 53 | 50 | 73 | 59 | 97% |
| S. aureus | 48 | 65 | 38 | 50 | 49 | 44 | 42 | 45 | 89% |
| *B. subtilis* | 53 | 61 | 53 | 56 | 25 | 20 | 22 | 22 | 40% |
| *S. choleraesuis* | 36 | 43 | 36 | 39 | 34 | 34 | 31 | 33 | 85% |
| *L. monocytogenes* | 43 | 38 | 22 | 34 | 26 | 31 | 34 | 30 | 88% |
| *C. albicans* | 36 | 25 | 21 | 27 | 20 | 12 | 27 | 20 | 72% |
| *S. mutans* | 11 | 19 | 13 | 14 | 9 | 16 | 14 | 13 | 91% |

### Example 11:Processed Morinda citrifolia as a Dental Medicament

Simple research may be performed to validate the development of *Morinda citrifolia* as a dental medicament. The present invention contemplates three areas of research that will confirm the safety and efficacy of the use of *Morinda citrifolia* as an oral care agent. First, reaseach will be done to examine the *in vitro* antimicrobial activity of *Morinda citrifolia* against common periodontal and endodontic pathogens. Second research will be done to examine the effectiveness of *Morinda citrifolia* to clean and disinfect root canals. Third, research will be done to assess the in-vitro biocompatibility of *Morinda citrifolia* to oral tissues.

The antimicrobial action of irrigants is tested using *in vitro* cultures of common periodontal and endodontic pathogens. The most widely accepted and utilized technique is to evaluate test materials using the standardized single disk method. This technique involves the inoculation of disks with test substances, in the present invention *Morinda citrifolia* to assess their antimicrobial activity. The advantage of using this standard technique is the ease of interpretation of antimicrobial activity and the high degree of reproducibility that can be accomplished. The effectiveness of test materials to clean and disinfect root canals can be evaluated using scanning electron microscopy to visualize the adherence and removal of microbes. The removal of microbes after endodontic therapy is important to avoid subsequent treatment failure due to complications created by the persistence of microbes. It is also important to remove any dentin smear layer to attempt to improve the sealing of endodontic materials with the tooth surfaces. The inadequate sealing of endodontic materials may allow the leakage of microbes into the oral tissues and this can create treatment complications leading to failure. If test materials prove to have an effective antimicrobial activity and are effective at cleaning and disinfecting root canals, they must be assessed for safety before they can be used as part of dental treatment. The toxicity of potential medicaments is examined using tests to measure biocompatibility. The interaction between tissues and materials is called biocompatibility. Biocompatibility is measured according to the assessment criteria defined by the International Organization for Standardization (ISO) and International Dental Federation. These organizations produced directives ISO 10993 and 7405 that specify the preclinical evaluation tests for devices and materials to be used as part of dental treatment. Dental materials that pass the ISO preclinical evaluation tests can be used worldwide as the standards incorporate local and national standards. The standards include the requirements of the U.S. Food and Drug Administration and American Dental Association.

The ideal irrigant should have an antimicrobial action, low toxicity and good biocompatibility to oral tissues, and have the capacity to clean the walls of the root canal and remove the smear layer. The smear layer is a 1 mm thick layer of denatured cutting debris produced on instrumented cavity surfaces, and is composed of dentin, odontohiastic processes, non-specific inorganic contaminants and microorganisms. The removal of smear layer from the instrumented root canal walls is controversial. Its removal provides better sealing of the endodontic filling material to dentin, and will avoid the leakage of microorganisms into oral tissues. The infiltration of microorganisms into oral tissues must be prevented because these often cause complications leading to treatment failure.

### 1. Examine the in vitro antimicrobial activity of Morinda citrifolia against common periodontal and endodontic pathogens.

The antimicrobial effects of *Morinda citrifolia* have not been tested on common periodontal and endodontic pathogens. The purpose of this part of the proposal is to evaluate the *in vitro* antimicrobial activity of *Morinda citrifolia* on seven commonly isolated oral microbial pathogens. Isolates from specific microbes will be obtained from the American Type Culture Collection (ATCC). The pure cultures will be stored in 1.5mL capped glass vials at -68°C to -72°C. After removing these vials from the freezer, they will be allowed to thaw before being inocuiated into sterile test tubes containing broth growth medium. The suspension will be agitated in a Vortex mixer and incubated for 3 to 5 days at 37° C in a Coy Anaerobic chamber containing an atmosphere of 85% nitrogen, 10% hydrogen and 5% carbon dioxide. For all the broth types, one control sample (no inoculum added) will be treated the same way as inoculated tubes to insure sterility.

After the inoculation period, the purity of each culture in the broth stage will be tested with gram stain microscopy before inoculation into agar plates. After agitating the cultures using a Vortex mixer, inoculation onto sheep blood agar plates will be performed by dripping sterile cotton swabs into the cultures, removing excess inoculate by pressing the swab against the side of the test tube, and then spreading the inoculum uniformly across the entire agar surface. The plates will be allowed to dry in air for 2 to 3 minutes.

The susceptibility of the various microorganis to *Morinda citrifolia* will be evaluated using the standardized single disk method similar to that employed to test for antibiotic sensitivity. Sterile blank disks will be carefully positioned on to the agar surface using sterile handling procedures. The disks will then be loaded with 10µl of various concentrations of *Morinda citrifolia.* Clindamycin disks will be used as a positive control, and sterile disks loaded with sterile saline instead of *Morinda citrifolia* will be used as a negative control. The disks will be spatially arranged on the surface of the blood agar as far apart from each other as possible (about 3cm), while maintaining a distance of at least 2cm from the edges of the Petri dish. The time elapsed between removing the culture suspension from the anaerobic chamber, spreading the inocula, placing the disks and loading the various experimental treatments is estimated to be 15 minutes. For each microbial species, five replicates of the three spatial arrangements of disks on agar plates will be prepared (15 plates per bacterial species). A concentrated solution of *Morinda citrifolia* will be diluted into ten-fold concentrations (dosages; 1x, 10x, 100x, and 1,000x) and loaded onto blank disks. The pH of all the *Morinda citrifolia* dilutions will be adjusted to be identical to the stock solution (e.g. pH 8.9).

The seeded plates will be incubated for 5 to 7 days in and anaerobic chamber. The zones of inhibition produced by the various concentrations of *Morinda citrifolia* will be measured using calipers. A reading of 7mm (diameter of the disk) indicates the absence of a zone of bacterial growth inhibition. The outer border of the zone of inhibition will be defined as the furthest concentric zone from the borders of the disk where complete bacterial growth inhibition occurs. In an effort to estimate the minimum inhibitory concentration of *Morinda citrifolia* for each of the 6 microbial species, intermediates doses of *Morinda citrifolia* will be calculated from concentrations that produced large zones of inhibition and those that did not inhibit bacterial growth. Five of each of these inhibitory doses will be performed.

All the data will be analyzed using a one way Analysis of Variance (ANOVA) statistical test, followed by the Scheffe post hoc multiple pairwise comparison test at a significance level of P<0.05. This statistical procedure is among the most conservative and reliable of the methods available to analyze raw data.

### 2. Examine the effectiveness of Morinda citrifolia to clean and disinfect root canals

Human teeth will be collected from clinics with Internal Review Board approval. Sixty freshly extracted, intact permanent teeth, which had not been stored in antibacterial or fixative solutions and had not received root canal medicaments will be selected. All teeth will have a single root canal, and the root lengths will range from 12 to 16mm. Conventional access preparations will be made and the root canals instrumented 1 mm beyond the apical forarmen with Hedstroem files up to size 50. Irrigation with sterile water will be performed during preparation. Following root canal preparation, the enlarged apical foramina will he sealed with epoxy resin to prevent bacterial leakage. To make handling and identification easier the teeth will be mounted vertically in plaster blocks and sterilized in an autoclave for 20 minutes at 121°C.

Pure culture of Enterococcus *faecalis* ATTCC 29212 grown in Brain Heart Infusion broth (BHF-Difco) will be used to contaminate the root canals within the extracted teeth. The root canals will be inoculated with 10 FL of a 1.5x10⁸ CFU mL suspension using sterile 1 mL tuberculin syringes. The plaster blocks will be placed inside stainless steel boxes and incubated at 37°C for 24 hours. After incubation the contaminated teeth will be divided into six groups according to the irrigation regimen used: (1) teeth (n1 0) with root canals irrigated with 2mL of *Morinda citrfolia* using an optimal concentration determined from phase 1 of this proposal; (2) teeth (n1 0) with root canals irrigated with 2mL of 2% chlorhexidine solution; (3) teeth (n1 0) with root canals irrigated with 2mL of 6% Sodium hypochorite solution; (4) teeth (n10) with root canals irrigated with 2 mL of 0.9% sterile saline solution as a negative control treatment; (5) teeth (n=10) with root canals irrigated with a 2mL mixture of *Morinda citrifolia* and 2% chiorhexidine solution; (6) teeth (n10) with root canals irrigated with a 2mL mixture of Morinda *citrifolia* and 6% Sodium hypochorite solution.

The 6 irrigation treatments (described above) will be delivered using a 2mL sterile plastic syringe and a 24 gauge needle. The needle will be inserted into the root canals approximately 1mm away from the walls, and 2mL of irrigant will be delivered. The irrigant will be placed in the canal for 5 minutes and removed, followed by irrigation with 1 mL of sterile saline solution.

The effectiveness of the 6 irrigation treatments to clean and disinfect the root canals will be assessed high-power micrograph images of the root canals using a scanning electron machine (SEM). The teeth will be prepared for use in the SEM by fixing the tooth tissues in 10% formalin solution at 18°C for 24 hours. The teeth will then be postfixed in osmium tetroxide (1%v/v) for 2 hours before being dehydrated in a graded series of ethanol solutions (20%, 50%, 70%, 90%, for 15 minutes each, followed by 3x 10 minutes of 100% ethanol). They will then be removed from the solutions and placed in hexamethyldistilazane for 5 minutes to fix the dehydrated specimens. The tooth slices will be dried on filter paper to dry. The dried specimens will be mounted onto aluminium stereoscan stubs with either conductive carbon cement or rapid set Araldite. The dried mounted specimens will be coated with a 20-30mm thin metallic layer of gold/palladium in a Poiaron E5000 sputter coater and viewed in a Quita 200 SEM. SEM micrographs will be obtained at x2,000 magnification using digital image analysis software. All 60 specimens (each split into two) will be examined and the micrograph images will be stored as digital files in an Acer Computer connected to the SEM. Each of the root canals will be scanned in its entirety to obtain an overview of the general surface topography. Micrographs will be taken of representative areas characteristic of the general surface topography of each specimen. The dentin root canal surfaces will be assessed for the presence of smear layer and microorganisms using a semi-quantitative visual criteria using the scale zero to four.

The effectiveness of *Morinda citrifolia* and irrigation treatments to clean and disinfect the root canals will be assessed using the semi-quantitative criteria:
0: no removal of microorganisms and smear layer from root canals.
1: less than 50% removal of microorganisms and smear layer.
2: approximately 50% removal of microorganisms and smear layer.
3: greater than 50% removal of microorganisms and smear layer but not complete removal.
4: complete removal of microorganisms and smear layer

### 3. Assess the in vitro biocompatibility of morinda citrifolia to oral tissues.

Toxicology studies were performed in rats to assess the acute toxicity of *Morinda citrifolia.* Fifteen thousand mg/kg was administered via gavage. The animals were observed for 14 d following treatment. All animals survived and no adverse clinical signs were noted. No signs of gross toxicity were seen in the organs after necropsy.

Two studies using guinea pigs were performed to assess the allergenic risk of *Morinda citrifolia.* Both study designs included an induction phase and a rest period, followed by a challenge with *Morinda citrifolia.* The first study involved two test groups of six animals each, a positive control group, and a negative control group. Following the challenge, the animals were observed for 24 h. No allergic reactions to *Morinda citrifolia* were seen in this study. The second study involved forty-five guinea pigs. The study consisted of several test groups using various forms and concentrations of *Morinda citrifolia* with accompanying negative control groups. The test groups were induced three times each week for two weeks. After thirty-two days of rest, all animals were challenged and were observed for symptoms of an allergic response. No positive allergic reactions were seen in any Noni group of the animals following the challenge.

A 13-week oral toxicity study in rats was performed to further assess the systemic safety of *Morinda citrifolia.* Eighty Sprague Dawley rats were allocated into four groups; a control group and three dose groups. The daily gavage doses included 0.4 mL/kg, 4 mL/kg, and 8 mL/kg. The animals were observed for adverse clinical signs, food consumption, and weight gain. Additionally, blood samples were drawn for hematology and clinical chemistry at the study conclusion. Further more, selective organ weights were measured and tissue samples of 55 organs were taken for microscopic examination. All groups showed no treatment related differences in body and organ weights, food consumption, clinical examinations, blood chemistry, hematological measurements, and histological tissue examination.

A second 13-week oral toxicity study of *Morinda citrifolia* was performed. This study covers higher doses than the previous 13-week study. Three dose groups were included in this study. The samples evaluated were a single strength *Morinda, citrifolia,* a 2.5 times concentrated *Morinda citrifolia,* and a 4 times concentrated *Morinda citrifolia.* The concentrated samples were used to reach a dosing equivalent of 50 mL/kg body weight and 80 mL/kg body weight. The protocol and measurements for the second 13-week study were the same as the first. The results of this study demonstrate that the No-Observable-Adverse-Effect-Level (NOAEL) was above 20 mL of 4 times concentrated *Morinda citrifolia* /kg/day. This is equivalent to 80 mL *Morinda citrifolia* /kg/day. Perspectively, this amount is 8% of the animal's body weight. No upper limit for safe consumption has yet been determined from these studies. The data indicates that *Morinda citrifolia* may be safely consumed in effective quantities.

Beyond the biocompatibility assays already performed there exists a number of screening tests that may be used to evaluate the safety of *Morinda citrifolia* before it can be used as a dental material. The screening tests for measuring the biocompatibility of dental materials are specified by the ISO. The ISO is a worldwide federation of national standards bodies (ISO member bodies). The ISO 7405 standards [international Standards Organization 2003] cover specifically dental materials and ISO 10993 standards [International Standards Organization 2003] cover specifically medical devices, which also include dental materials. The international standards is a harmonization and combination of national standards including American Dental Association ANSI-ADA document number 41 [Acceptance program guidelines, 1998; American Dental Association, 1997], British Standard 5736 [1989], and Deutches Institut fur Normung DIN V 13 930 [1996]. The ISO defines a dental material as a substance or combination of substances specially prepared and/or presented for use by authorized persons in the practice of dentistry and/or It's associated procedures. The stated primary goal of ISO 10993 entitled the Biological evaluation of medical devices is the protection of humans [International Standards Organization, 2003]. It is the most up to date overall guidance document for the selection of tests, to be used for the evaluation of biological responses relevant to medical or dental material and device safety. ISO 7405 is entitled the Preclinical evaluation of biocompatibility of medical devices used in Dentistry - Test methods for Dental Materials [International Standards Organization, 2003] it concerns the preclinical testing of materials used in dentistry and supplements ISO 10993.

Guidelines ISO 10993 and ISO 7405 have recommended standard practices for the biological evaluation of dental materials. In summary these include; (i) It is incumbent upon the dental material manufacturer to select the appropriate tests, based on the intended use of the material, and known or assumed toxicity profile of the material or its components. (ii) A manufacturer may select one of three cytotoxicity tests in preference to another because of cost, experience or other reasons. (iii) New materials should be evaluated using initial cytotoxicity and secondary tissue screening tests prior to extensive animal experimentation and clinical trials. (iv) Test results should always be interpreted with consideration for the manufacturers stated use for the material.

To accomplish phase II of this research proposal we intend to evaluate *Morinda citrifolia* according to the first phase of the biological testing program recommended by the ISO. Provided these tests are successful. We will then conduct the other preclinical phases of the biological testing program in a subsequent follow-up research.

Mouse fibroblast permanent cell line (clone L-929) has provided a means of good reproducibility of cytotoxicity testing between different laboratories and materials. This cell line is easy to grow and it has been widely used for the biological screening of dental materials. The L-929 cell line will be grown to confluence in 50 ml culture dishes using Dulbeccos Modified Eagles Medium (VWR, Suwanee, GA, USA), 10% Fetal Bovine Serum (VWR, Suwanee, GA, USA), and 1% penicillin and streptomysin (VWR, Suwanee, GA, USA). The cell cultures will be maintained in a 5% carbon dioxide incubator at 37° C throughout the testing period. Immediately Prior to testing, the cells will be stained with a neutral red vital dye stain (VWR, Suwanee, GA USA). A thin layer of agar will be prepared and placed on top of the cell cultures using a sterile technique. A cellulose acetate milipore filter is placed in contact with the confluent cell cultures. A concentrated solution of *Morinda citrifolia* will be diluted into ten-fold concentrations (dosages; 1x, 10x, 100x, and 1,000X) and 50 µl will be loaded onto the filters. For each concentration, five replicates will be prepared. The testing period will be 24 hours. During this time any leachable substances must diffuse through the 0.45 µm filter pores to exert any cytotoxic effects on the cells. The appearance of the test filters between the *Morinda citrifolia*-cell contact areas will be registered according to a scoring system to classify the cytotoxic response. The presence of leachable toxic substances from *Morinda citrifolia* will be manifested by a loss of dye within the cells as they lyse. The ability of this simple strategy to determine the cytotoxic hazard of a material *in vitro* and generalize this to *in vivo* systems has been successful because the results are easy to interpret.

Clauses specifying aspects of the invention are set out below.
1. A composition for promoting oral care comprising:
   an effective amount of processed *Morinda citrifolia* product wherein said *Morinda citrifolia* is selected from the group consisting of *Morinda citrifolia* fruit, *Morinda citrifolia* fruit extract, *Morinda citrifolia* fruit juice concentrate, *Morinda citrifolia* oil, *Morinda citrifolia* leaf powder, and *Morinda citrifolia* leaf extract; and
   a carrier.
2. The composition of clause 1, wherein the carrier is comprised of ingredients selected from the group consisting of emulsifier, alcohol, sweeteners, thickening agents, surfactants, suspending agents, astringent and toning drug extracts, flavor correctants, abrasives or polishes, deodorizing agents, preservatives, flavoring buffers, whitening agents, wound-healing and inflammation inhibiting substances, colorants, dyes, pigments, abrasives, polishes, antimicrobial agents, clouding agents, gelling agents, a prophylaxis paste, pH buffers and combinations thereof.
3. The composition of clause 1, wherein the carrier further comprises substances selected from the group consisting of glycerin, edible polyhydric alcohols, polyols and combinations thereof.
4. The composition of clause 3, wherein the polyols are selected from the group consisting of glycerol, propylene glycol, propylene glycol glycerol, polyethylene glycol, isomalt, xylitol, maltitol, sorbitol, mannitol and combinations thereof.
5. The composition of clause 1, wherein the carrier is comprised of flavoring agents selected from the group of compounds consisting of oil of peppermint, oil of wintergreen, oil of spearmint, clove bud oil, parsley oil, eucalyptus oil, menthol, menthane, anethole, methyl salicylate, eucalyptol, cassia, 1-methyl acetate, sage, eugenol, oxanone, alpha-irisone, marjoram, lemon, orange, propenyl guaethol acetyl, cinnamon, vanilla, thymol, linalool, cinnamaldehyde glycerol acetal, N-substituted p-menthane-3-carboxamides, 3,1-methoxy propane 1,2-diol and combinations thereof.
6. The composition of clause 1, wherein the carrier is further comprised of active compounds selected from the group consisting of chlorine dioxide, fluoride, alcohols, triclosan, domiphen bromide, cetyl pridinium chlorine, calcium lactate, calcium lactate salts, sodium fluoride, stannous fluoride, sodium monofluorophosphate, cetyl pyridium chloride, zinc salts, pyrophosphate, 1-hydroxyethane-1,2-diphosphonic acid, 1-phosphonopropane-1,2,3-tricarbox- ylic acid, azacycloalkane-2,2-diphosphonic acids, cyclic aminophosphonic acids and combinations thereof.
7. The composition of clause 1, wherein the composition is in the form of toothpaste, gel, mouthwash, mouth rinse, gum, mouth spray, lozenge, subgingival irrigation fluid, coated fiber floss, toothbrush bristle, an interproximal dental brush, a topically applied solution, candy gum drops, candy pieces, candy bars, and nougats or combinations thereof.
8. The compositions of clause 2, wherein the sweetener is selected from the group consisting of saccharin, fructose, xylitol, saccharin salts, thaumatin, aspartame, D-tryptophan, dihydrochalcones, acesulfame, cyclamate salts, and combinations thereof.
9. The compositions of clause 2, wherein the clouding agents are selected from the group consisting of calcium citrate, esters of wood rosin, vegetable gum emulsion, caprylic/capric triglycerides, guar gum, gum arabic and high-stability oils.
10. The compositions of clause 2, wherein the abrasives or polishes are selected from the group consisting of chalk, calcium carbonate, dicalcium phosphate, aluminum silicates, calcium pyrophosphate, finely particulate synthetic resins, silicas, aluminum oxide, aluminum oxide trihydrate, hydroyapatite finely insoluble sodium metaphosphate particulate, xerogel silica, hydrogel silica, precipitated silica, aluminum oxide trihydrate and finely particulate aluminum oxide and combinations thereof.
11. The compositions of clause 2, wherein the preservatives and antimicorbial agents are selected from the group consisting of p-hydroxybenzoic acid, methyl ester, ethyl ester, propyl ester, sodium sorbate, sodium benzoate, bromochlorophene, phenyl salicylic acid esters, thymol, and combinations thereof.
12. The compositions of clause 2, wherein the pH buffers are selected from the group consisting of primary, secondary or tertiary alkali phosphates, citric acid, sodium citrate and combinations thereof.
13. The compositions of clause 1, wherein the carrier is comprised of wound healing and inflammation inhibiting substances selected from the group consisting of allantoin, urea, azulene, camomile active substances, acetyl salicylic acid derivatives and combinations thereof.
14. The composition of clause 2, wherein said gelling agent is selected from the group consisting of polycarboxylic acids, polycarboxylic acid salts, polysaccharides, polysaccharide derivatives, proteins, protein derivatives, polyalkylene oxides and fumed silica.
15. The composition of clause 1, wherein said effective amount of *Morinda citrifolia* comprises between about 8 and 33 percent by composition weight, and wherein said carrier comprises between about 75 and 90 percent by composition weight.
16. The composition of clause 1, wherein said effective amount of *Morinda citrifolia* comprises between about 5 and 25 percent by composition weight, and wherein said carrier comprises glycerine, calcium carbonate, and silica.
17. The composition of clause 16, wherein said glycerine comprising between 10 and 50 percent by carrier weight, said calcium carbonate comprising between 20 and 40 percent by carrier weight, and said silica comprising between about 20 and 40 percent by carrier weight.
18. A method for treating and preventing oral and dental disorders, said method comprising:
   providing an effective amount of a *Morinda citrifolia* product, wherein said *Morinda citrifolia* product is selected from the group consisting of *Morinda citrifolia* fruit, *Morinda citrifolia* fruit extract, *Morinda citrifolia* fruit juice concentrate, *Morinda citrifolia* oil, *Morinda citrifolia* leaf powder, and *Morinda citrifolia* leaf extract.;
   combining a carrier with said effective amount of *Morinda citrifolia* to produce an oral care composition;
   administering and effective amount of said oral care composition to a patient.
19. The method of clause 18, wherein said administering said oral care composition further comprises topically applying said oral care composition to at least of one of said patient's teeth, gums and surrounding oral tissues.
20. The method of clause 18, wherein said administering said oral care composition further comprises orally administering to said patient said oral care composition as a dietary supplement.
21. The method of clause 18, wherein the carrier is comprised of ingredients selected from the group consisting of emulsifier, alcohol, sweeteners, thickening agents, surfactants, suspending agents, astringent and toning drug extracts, flavor correctants, abrasives or polishes, deodorizing agents, preservatives, flavoring buffers, whitening agents, wound-healing and inflammation inhibiting substances, colorants, dyes, pigments, abrasives, polishes, antimicrobial agents, clouding agents, gelling agents, prophylaxis paste, pH buffers, glycerin, edible polyhydric alcohols, polyols and combinations thereof.
22. The method of clause 21, wherein the polyols are selected from the group consisting of glycerol, propylene glycol, propylene glycol glycerol, polyethylene glycol, isomalt, xylitol, maltitol, sorbitol, mannitol and combinations thereof.
23. The method of clause 18, wherein the carrier is comprised of flavoring agents selected from the group of compounds consisting of oil of peppermint, oil of wintergreen, oil of spearmint, clove bud oil, parsley oil, eucalyptus oil, menthol, menthane, anethole, methyl salicylate, eucalyptol, cassia, 1-methyl acetate, sage, eugenol, oxanone, alpha-irisone, marjoram, lemon, orange, propenyl guaethol acetyl, cinnamon, vanilla, thymol, linalool, cinnamaldehyde glycerol acetal, N-substituted p-menthane-3-carboxamides, 3,1-methoxy propane 1,2-diol and combinations thereof.
24. The method of clause 21, wherein the carrier is further comprised of active compounds selected from the group consisting of chlorine dioxide, fluoride, alcohols, triclosan, domiphen bromide, cetyl pridinium chlorine, calcium lactate, calcium lactate salts, sodium fluoride, stannous fluoride, sodium monofluorophosphate, cetyl pyridium chloride, zinc salts, pyrophosphate, 1-hydroxyethane-1,2-diphosphonic acid, 1-phosphonopropane-1,2,3-tricarbox- ylic acid, azacycloalkane-2,2-diphosphonic acids, cyclic aminophosphonic acids and combinations thereof.
25. The method of clause 18, wherein the composition is in the form of toothpaste, gel, mouthwash, mouth rinse, gum, mouth spray, lozenge, subgingival irrigation fluid, coated fiber floss, toothbrush bristle, an interproximal dental brush, a topically applied solution, candy gum drops, candy pieces, candy bars, and nougats or combinations thereof.
26. The method of clause 21, wherein the sweetener is selected from the group consisting of saccharin, fructose, xylitol, saccharin salts, thaumatin, aspartame, D-tryptophan, dihydrochalcones, acesulfame, cyclamate salts, and combinations thereof.
27. The method of clause 21, wherein the clouding agents are selected from the group consisting of calcium citrate, esters of wood rosin, vegetable gum emulsion, caprylic/capric triglycerides, guar gum, gum arabic and high-stability oils.
28. The method of clause 21, wherein the abrasives or polishes selected from the group consisting of chalk, calcium carbonate, dicalcium phosphate, aluminum silicates, calcium pyrophosphate, finely particulate synthetic resins, silicas, aluminum oxide, aluminum oxide trihydrate, hydroyapatite finely insoluble sodium metaphosphate particulate, xerogel silica, hydrogel silica, precipitated silica, aluminum oxide trihydrate and finely particulate aluminum oxide and combinations thereof.
29. The methods of clause 21, wherein the preservatives and antimicorbial agents are selected from the group consisting of p-hydroxybenzoic acid, methyl ester, ethyl ester, propyl ester, sodium sorbate, sodium benzoate, bromochlorophene, phenyl salicylic acid esters, thymol, and combinations thereof.
30. The methods of clause 21, wherein the pH buffers are selected from the group consisting of primary, secondary or tertiary alkali phosphates, citric acid, sodium citrate and combinations thereof.
31. The methods of clause 21, wherein the wound healing and inflammation inhibiting substances are selected from the group consisting of allantoin, urea, azulene, camomile active substances, acetyl salicylic acid derivatives and combinations thereof.
32. The method of clause 21, wherein said gelling agent is selected from the group consisting of polycarboxylic acids, polycarboxylic acid salts, polysaccharides, polysaccharide derivatives, proteins, protein derivatives, polyalkylene oxides and fumed silica.
33. The method of clause 18, wherein said effective amount of *Morinda citrifolia* comprises between about 8 and 33 percent by composition weight, and wherein said carrier comprises between about 75 and 90 percent by composition weight.
34. The method of clause 18, wherein said effective amount of *Morinda citrifolia* between about 10 and 50 percent by composition weight, and wherein said carrier comprises between about 48 and 88 percent by composition weight.
35. The method of clause 18, wherein said effective amount of *Morinda citrifolia* comprises between about 5 and 25 percent by composition weight, and wherein said carrier comprises glycerine, calcium carbonate, and silica.
36. The method of clause 35, wherein said glycerine comprising between 10 and 50 percent by carrier weight, said calcium carbonate comprising between 20 and 40 percent by carrier weight, and said silica comprising between about 20 and 40 percent by carrier weight.

## Claims

1. A composition for use in treating tooth decay, the composition comprising:
an effective amount of processed *Morinda citrifolia* product wherein said *Morinda citrifolia* is selected from the group consisting of *Morinda citrifolia* fruit, *Morinda citrifolia* fruit extract, *Morinda citrifolia* fruit juice concentrate, *Morinda citrifolia* oil, *Morinda citrifolia* leaf powder, and *Morinda citrifolia* leaf extract; and
a carrier.

2. The composition of claim 1, wherein the carrier is comprised of ingredients selected from the group consisting of emulsifier, alcohol, sweeteners, thickening agents, surfactants, suspending agents, astringent and toning drug extracts, flavor correctants, abrasives or polishes, deodorizing agents, preservatives, flavoring buffers, whitening agents, wound-healing and inflammation inhibiting substances, colorants, dyes, pigments, abrasives, polishes, antimicrobial agents, clouding agents, gelling agents, a prophylaxis paste, pH buffers and combinations thereof.

3. The composition of claim 1, wherein the carrier further comprises substances selected from the group consisting of glycerin, edible polyhydric alcohols, polyols and combinations thereof.

4. The composition of claim 3, wherein the polyols are selected from the group consisting of glycerol, propylene glycol, propylene glycol glycerol, polyethylene glycol, isomalt, xylitol, maltitol, sorbitol, mannitol and combinations thereof.

5. The composition of claim 1, wherein the carrier is comprised of flavoring agents selected from the group of compounds consisting of oil of peppermint, oil of wintergreen, oil of spearmint, clove bud oil, parsley oil, eucalyptus oil, menthol, menthane, anethole, methyl salicylate, eucalyptol, cassia, 1-methyl acetate, sage, eugenol, oxanone, alpha-irisone, marjoram, lemon, orange, propenyl guaethol acetyl, cinnamon, vanilla, thymol, linalool, cinnamaldehyde glycerol acetal, N-substituted p-menthane-3-carboxamides, 3,1-methoxy propane 1,2-diol and combinations thereof.

6. The composition of claim 1, wherein the carrier is further comprised of active compounds selected from the group consisting of chlorine dioxide, fluoride, alcohols, triclosan, domiphen bromide, cetyl pridinium chlorine, calcium lactate, calcium lactate salts, sodium fluoride, stannous fluoride, sodium monofluorophosphate, cetyl pyridium chloride, zinc salts, pyrophosphate, 1-hydroxyethane-1,2-diphosphonic acid, 1-phosphonopropane-1,2,3-tricarbox- ylic acid, azacycloalkane-2,2-diphosphonic acids, cyclic aminophosphonic acids and combinations thereof.

7. The composition of claim 1, wherein the composition is in the form of toothpaste, gel, mouthwash, mouth rinse, gum, mouth spray, lozenge, subgingival irrigation fluid, coated fiber floss, toothbrush bristle, an interproximal dental brush, a topically applied solution, candy gum drops, candy pieces, candy bars, and nougats or combinations thereof.

8. The compositions of claim 2, wherein the preservatives and antimicrobial agents are selected from the group consisting of p-hydroxybenzoic acid, methyl ester, ethyl ester, propyl ester, sodium sorbate, sodium benzoate, bromochlorophene, phenyl salicylic acid esters, thymol, and combinations thereof.

9. The compositions of claim 2, wherein the pH buffers are selected from the group consisting of primary, secondary or tertiary alkali phosphates, citric acid, sodium citrate and combinations thereof.

10. The compositions of claim 1, wherein the carrier is comprised of wound healing and inflammation inhibiting substances selected from the group consisting of allantoin, urea, azulene, camomile active substances, acetyl salicylic acid derivatives and combinations thereof.

11. The composition of claim 2, wherein said gelling agent is selected from the group consisting of polycarboxylic acids, polycarboxylic acid salts, polysaccharides, polysaccharide derivatives, proteins, protein derivatives, polyalkylene oxides and fumed silica.

12. The composition of claim 1, wherein said effective amount of *Morinda citrifolia* comprises between 8 and 33 percent by composition weight, and wherein said carrier comprises between 75 and 90 percent by composition weight.

13. The composition of claim 1, wherein said effective amount of *Morinda citrifolia* comprises between about 5 and 25 percent by composition weight, and wherein said carrier comprises glycerine, calcium carbonate, and silica.

14. The composition of claim 13, wherein said glycerine comprising between 10 and 50 percent by carrier weight, said calcium carbonate comprising between 20 and 40 percent by carrier weight, and said silica comprising between 20 and 40 percent by carrier weight.

15. The use of processed *Morinda citrifolia* product in the manufacture of a medicament for the treatment of tooth decay; wherein said *Morinda citrifolia* is selected from the group consisting of *Morinda citrifolia* fruit, *Morinda citrifolia* fruit extract, *Morinda citrifolia* fruit juice concentrate, *Morinda citrifolia* oil, *Morinda citrifolia* leaf powder, and *Morinda citrifolia* leaf extract.
